# EUROPEAN PATENT APPLICATION

(11) **EP 3 961 638 A1**
(43) Date of publication of application: **02.03.2022**
(21) Application number: 20795717.6
(22) Date of filing: 24.04.2020
(51) Int. Cl.: G16B 30/20

(54) **NOVEL METHOD FOR PROCESSING SEQUENCE INFORMATION ABOUT SINGLE BIOLOGICAL UNIT**

(30) Priority: 26.04.2019 JP 2019085839
(71) Applicant: bitBiome, Inc., Tokyo 169-0051 (JP)
(72) Inventor: ARIKAWA Koji, Tokyo 169-8050 (JP); HOSOKAWA Masahito, Tokyo 169-8050 (JP); TAKEYAMA Haruko, Tokyo 169-8050 (JP); KOGAWA Masato, Tokyo 169-8050 (JP); IDE Keigo, Tokyo 169-8050 (JP)
(74) Representative: Uexküll & Stolberg
(86) International application number: PCT/JP2020/017795
(87) International publication number: WO 2020/218555

(57) **Abstract**

The present disclosure provides a system for automatically constructing and providing microbial genome data. This method for processing sequence information about a single biological unit includes: (A) a step for performing clustering, for each same lineage, on partial sequence information (in a slide, SAG) about a genome (or an equivalent gene set) of a plurality of single biological units (for example, cells), on the basis of a sequence (16S rRNA or a marker gene) for identifying biological lineage; and (B) a step for performing collation with information about a genome of the single biological units in a database, if necessary.

## Description

### [Technical Field]

The present disclosure provides a novel method, system, and related technology for processing sequence information on a single biological unit. More specifically, the present disclosure provides a system for automatically constructing and providing microorganism genomic data.

### [Background Art]

While construction of microorganism genomic data is advancing, current data is often based on metagenomic information. This is insufficient in terms of quality and quantity as information when targeting the analysis on complex bacterial flora.

Although some genetic information (genomic information, etc.) is obtained for each single biological unit, information processing thereof with sufficient quality has not been provided.

### [Summary of Invention]

### [Solution to Problem]

As a result of diligent studies, the inventors have completed a system for accumulating sequence information on a single biological unit at a single biological unit level and automatically constructing and providing highly accurate microorganism genomic data therefrom.

Examples of embodiments of the present disclosure include the following.
(Item 1) A method of processing sequence information on a single biological unit, the method comprising:
   (A) a step of clustering partial sequence information of sequence information on a plurality of single biological units for each of the same lineages based on an organism lineage identification sequence;
   (B) optionally, a step of adding, to the cluster, partial sequence information on the single biological units corresponding to the cluster in a database; and
   (C) a step of creating a sequence information draft for the single biological units by using the partial sequence information of sequence information on the single biological units and sequence information on the single biological units in the database.
(Item 2) The method of item 1, further comprising utilizing a database if step (B) is performed.
(Item 3) A method of processing sequence information on a single biological unit, the method comprising:
   A) a step of extracting genes without duplication from a draft in a database;
   B) a step of calculating the number or a ratio of corresponding drafts for each of the genes; and
   C) a step of selecting a gene with the number or ratio of the corresponding drafts greater than or equal to a predetermined value as a candidate of an organism lineage identification sequence.
(Item 3A) A method of processing sequence information on a single biological unit, the method comprising:
   (A) a step of clustering partial sequence information of sequence information on a plurality of single biological units for each of the same lineages based on an organism lineage identification sequence; and
   (B) a step of comparing partial sequence information corresponding to the cluster in a database with partial sequence information of the cluster, calculating a degree of similarity for each partial sequence, and identifying a partial sequence with a degree of similarity greater than or equal to a predetermined degree of similarity as an organism lineage identification sequence.
(Item 4) A method of processing sequence information on a single biological unit, the method comprising:
   (D) a step of ranking partial sequence information of sequence information on a plurality of single biological units from a highest quality based on a predetermined judgement criterion;
   (E) a step of selecting a population of a predetermined number of pieces of partial sequence information of sequence information on the plurality of single biological units from a highest ranking based on the ranking to construct a draft with a greater length than the partial sequence information from the partial sequence information; and
   (E') a step of selecting a population of a set of a different number of pieces of partial sequence information of sequence information on the single biological units from the population, constructing a draft with a greater length than the partial sequence information from the partial sequence information, and selecting a draft created up to this point based on a predetermined judgment criterion.
(Item 5) The method of processing sequence information on a single biological unit of item 4, the method comprising:
   (F) a step of comparing the selected draft with partial sequence information of sequence information on a single biological unit that was not selected in (E) or (E') and selecting partial sequence information of sequence information on the single biological unit having a sequence of a portion that is not included in the draft;
   (G) a step of creating a longer draft by using the sequence information selected in (F) and the selected draft;
   (G') optionally, a step of repeating (G) until the longer draft reaches a full length of sequence information; and
   (G") optionally, a step of repeating the steps of item 4 based on a judgment criterion with a lower criterion in the entire partial sequence information constituting the draft.
(Item 6) A method of processing sequence information on a single biological unit, the method comprising:
   (A) a step of clustering partial sequence information of sequence information on a plurality of single biological units for each of the same lineages based on an organism lineage identification sequence;
   (H) a step of evaluating partial sequence information of sequence information on the plurality of single biological units constituting sequence information on a single biological unit based on an organism lineage identification sequence within the cluster of the same lineage, and reclustering within the cluster of the same lineage;
   (H') a step of comparing a sequence information draft created from the cluster of the same lineage with a sequence information draft created from the reclustered cluster; and (I) a step of determining whether reclustering in (H) is appropriate for a result of comparison based on a predetermined judgment criterion, and, if appropriate, registering the draft in a database as a new group.
(Item 7) The method of item 6, wherein the reclustering is performed through network analysis and community detection.
(Item 8) A method of processing sequence information on a single biological unit, the method comprising:
   (A) a step of clustering partial sequence information of sequence information on a plurality of single biological units for each of the same lineages based on an organism lineage identification sequence;
   (D) a step of ranking partial sequence information of sequence information on a plurality of single biological units belonging to the cluster of the same lineage from a highest quality based on a predetermined judgement criterion;
   (E) a step of selecting a population of a predetermined number of pieces of partial sequence information of sequence information on the plurality of single biological units from a highest ranking based on the ranking to construct a draft with a greater length than the partial sequence information from the partial sequence information;
   (E") a step of selecting a population of a set of a different number of pieces of partial sequence information of sequence information on the single biological unit from the population, constructing a draft with a greater length than the partial sequence information from the partial sequence information, and evaluating a draft created up to this point based on a predetermined judgment criterion;
   (H) a step of evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change due to an increase in the number in a population of a set of sequence information;
   (H') a step of comparing a sequence information draft created from the cluster of the same lineage with a sequence information draft created from the reclustered cluster; and (J) a step of determining whether reclustering in (H) is appropriate for a result of comparison based on a predetermined judgment criterion, and, if appropriate, repeating (D) to (E') for partial sequence information of sequence information on the plurality of single biological units belonging to the reclustered cluster.
(Item 9) A program for implementing a method of processing sequence information on a single biological unit on a computer, the method comprising:
   (A) a step of clustering partial sequence information of sequence information on a plurality of single biological units for each of the same lineages based on an organism lineage identification sequence;
   (B) optionally, a step of adding, to the cluster, partial sequence information on the single biological units corresponding to the cluster in a database; and
   (C) a step of creating a sequence information draft for the single biological units by using the partial sequence information of sequence information on the single biological units and sequence information on the single biological units in the database.
(Item 10) The program of item 9, further comprising utilizing a database if step (B) is performed.
(Item 11) A program for implementing a method of processing sequence information on a single biological unit on a computer, the method comprising:
   A) a step of extracting genes without duplication from a draft in a database;
   B) a step of calculating the number or a ratio of corresponding drafts for each of the genes; and
   C) a step of selecting a gene with the number or ratio of corresponding drafts greater than or equal to a predetermined value as a candidate of an organism lineage identification sequence.
(Item 11A) A program for implementing a method of processing sequence information on a single biological unit on a computer, the method comprising:
   (A) a step of clustering partial sequence information of sequence information on a plurality of single biological units for each of the same lineages based on an organism lineage identification sequence; and
   (B) a step of comparing partial sequence information corresponding to the cluster in a database with partial sequence information of the cluster, calculating a degree of similarity for each partial sequence, and identifying a partial sequence with a degree of similarity greater than or equal to a predetermined degree of similarity as an organism lineage identification sequence.
(Item 12) A program for implementing a method of processing sequence information on a single biological unit on a computer, the method comprising:
   (D) a step of ranking partial sequence information of sequence information on a plurality of single biological units from a highest quality based on a predetermined judgment criterion;
   (E) a step of selecting a population of a predetermined number of pieces of partial sequence information of sequence information on the plurality of single biological units from a highest ranking based on the ranking to construct a draft with a greater length than the partial sequence information from the partial sequence information; and
   (E') a step of selecting a population of a set of a different number of pieces of partial sequence information of sequence information on the single biological units from the population, constructing a draft with a greater length than the partial sequence information from the partial sequence information, and selecting a draft created up to this point based on a predetermined judgment criterion.
(Item 13) The program for implementing a method of processing sequence information on a single biological unit on a computer of item 12, the method comprising:
   (F) a step of comparing the selected draft with partial sequence information of sequence information on the single biological unit that was not selected in (E) or (E') and selecting partial sequence information of sequence information on the single biological unit having a sequence of a portion that is not included in the draft;
   (G) a step of creating a longer draft by using the sequence information selected in (F) and the selected draft;
   (G') optionally, a step of repeating (G) until the longer draft reaches a full length of sequence information; and
   (G") optionally, a step of repeating the steps of item 12 based on a judgment criterion with a lower criterion in the entire partial sequence information constituting the draft.
(Item 14) A program for implementing a method of processing sequence information on a single biological unit on a computer, the method comprising:
   (A) a step of clustering partial sequence information of sequence information on a plurality of single biological units for each of the same lineages based on an organism lineage identification sequence;
   (H) a step of evaluating partial sequence information of sequence information on the plurality of single biological units constituting sequence information on a single biological unit based on an organism lineage identification sequence within the cluster of the same lineage, and reclustering within the cluster of the same lineage;
   (H') a step of comparing a sequence information draft created from the cluster of the same lineage with a sequence information draft created from the reclustered cluster; and (I) a step of determining whether reclustering in (H) is appropriate for a result of comparison based on a predetermined judgment criterion, and, if appropriate, registering the draft in a database as a new group.
(Item 15) The program of item 14, wherein the reclustering is performed through network analysis and community detection.
(Item 16) A program for implementing a method of processing sequence information on a single biological unit on a computer, the method comprising:
   (A) a step of clustering partial sequence information of sequence information on a plurality of single biological units for each of the same lineages based on an organism lineage identification sequence;
   (D) a step of ranking partial sequence information of sequence information on a plurality of single biological units belonging to the cluster of the same lineage from a highest quality based on a predetermined judgment criterion;
   (E) a step of selecting a population of a predetermined number of pieces of partial sequence information of sequence information on the plurality of single biological units from a highest ranking based on the ranking to construct a draft with a greater length than the partial sequence information from the partial sequence information;
   (E") a step of selecting a population of a set of a different number of pieces of partial sequence information of sequence information on the single biological unit from the population, constructing a draft with a greater length than the partial sequence information from the partial sequence information, and evaluating a draft created up to this point based on a predetermined judgment criterion;
   (H) a step of evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change due to an increase in the number in a population of a set of sequence information;
   (H') a step of comparing a sequence information draft created from the cluster of the same lineage with a sequence information draft created from the reclustered cluster; and (J) a step of determining whether reclustering in (H) is appropriate for a result of comparison based on a predetermined judgment criterion, and, if appropriate, repeating (D) to (E') for partial sequence information of sequence information on the plurality of single biological units belonging to the reclustered cluster.
(Item 17) A recording medium storing a program for implementing a method of processing sequence information on a single biological unit on a computer, the method comprising:
   (A) a step of clustering partial sequence information of sequence information on a plurality of single biological units for each of the same lineages based on an organism lineage identification sequence;
   (B) optionally, a step of adding, to the cluster, partial sequence information on the single biological units corresponding to the cluster in a database; and
   (C) a step of creating a sequence information draft for the single biological units by using the partial sequence information of sequence information on the single biological units and sequence information on the single biological units in the database.
(Item 18) The recording medium of item 17, further comprising utilizing a database if step (B) is performed.
(Item 19) A recording medium storing a program for implementing a method of processing sequence information on a single biological unit on a computer, the method comprising:
   A) a step of extracting genes without duplication from a draft in a database;
   B) a step of calculating the number or a ratio of corresponding drafts for each of the genes; and
   C) a step of selecting a gene with the number or ratio of corresponding drafts greater than or equal to a predetermined value as a candidate of an organism lineage identification sequence.
(Item 19A) A recording medium storing a program for implementing a method of processing sequence information on a single biological unit on a computer, the method comprising:
   (A) a step of clustering partial sequence information of sequence information on a plurality of single biological units for each of the same lineages based on an organism lineage identification sequence; and
   (B) a step of comparing partial sequence information corresponding to the cluster in a database with partial sequence information of the cluster, calculating a degree of similarity for each partial sequence, and identifying a partial sequence with a degree of similarity greater than or equal to a predetermined degree of similarity as an organism lineage identification sequence.
(Item 20) A recording medium storing a program for implementing a method of processing sequence information on a single biological unit on a computer, the method comprising:
   (D) a step of ranking partial sequence information of sequence information on a plurality of single biological units from a highest quality based on a predetermined judgment criterion;
   (E) a step of selecting a population of a predetermined number of pieces of partial sequence information of sequence information on the plurality of single biological units from a highest ranking based on the ranking to construct a draft with a greater length than the partial sequence information from the partial sequence information; and
   (E') a step of selecting a population of a set of a different number of pieces of partial sequence information of sequence information on the single biological units from the population, constructing a draft with a greater length than the partial sequence information from the partial sequence information, and selecting a draft created up to this point based on a predetermined judgment criterion.
(Item 21) The recording medium storing a program for implementing a method of processing sequence information on a single biological unit on a computer of item 20, the method comprising:
   (F) a step of comparing the selected draft with partial sequence information of sequence information on a single biological unit that was not selected in (E) or (E') and selecting partial sequence information of sequence information on the single biological unit having a sequence of a portion that is not included in the draft;
   (G) a step of creating a longer draft by using the sequence information selected in (F) and the selected draft;
   (G') optionally, a step of repeating (G) until the longer draft reaches a full length of sequence information; and
   (G") optionally, a step of repeating the steps of item 20 based on a judgment criterion with a lower criterion in the entire partial sequence information constituting the draft.
(Item 22) A recording medium storing a program for implementing a method of processing sequence information on a single biological unit on a computer, the method comprising:
   (A) a step of clustering partial sequence information of sequence information on a plurality of single biological units for each of the same lineages based on an organism lineage identification sequence;
   (H) a step of evaluating partial sequence information of sequence information on the plurality of single biological units constituting sequence information on a single biological unit based on an organism lineage identification sequence within the cluster of the same lineage, and reclustering within the cluster of the same lineage;
   (H') a step of comparing a sequence information draft created from the cluster of the same lineage with a sequence information draft created from the reclustered cluster; and (I) a step of determining whether reclustering in (H) is appropriate for a result of comparison based on a predetermined judgment criterion, and, if appropriate, registering the draft in a database as a new group.
(Item 23) The recording medium of item 22, wherein the reclustering is performed through network analysis and community detection.
(Item 24) A recording medium storing a program for implementing a method of processing sequence information on a single biological unit on a computer, the method comprising:
   (A) a step of clustering partial sequence information of sequence information on a plurality of single biological units for each of the same lineages based on an organism lineage identification sequence;
   (D) a step of ranking partial sequence information of sequence information on the plurality of single biological units belonging to the cluster of the same lineage from a highest quality based on a predetermined judgment criterion;
   (E) a step of selecting a population of a predetermined number of pieces of partial sequence information of sequence information on the plurality of single biological units from a highest ranking based on the ranking to construct a draft with a greater length than the partial sequence information from the partial sequence information; and
   (E") a step of selecting a population of a set of a different number of pieces of partial sequence information of sequence information on the single biological unit from the population, constructing a draft with a greater length than the partial sequence information from the partial sequence information, and evaluating a draft created up to this point based on a predetermined judgment criterion;
   (H) a step of evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change due to an increase in the number in a population of a set of sequence information;
   (H') a step of comparing a sequence information draft created from the cluster of the same lineage with a sequence information draft created from the reclustered cluster; and (J) a step of determining whether reclustering in (H) is appropriate for a result of comparison based on a predetermined judgment criterion, and, if appropriate, repeating (D) to (E') for partial sequence information of sequence information on the plurality of single biological units belonging to the reclustered cluster.
(Item 25) A system for processing sequence information on a single biological unit, the system comprising:
   (A) a clustering unit for clustering partial sequence information of sequence information on a plurality of single biological units for each of the same lineages based on an organism lineage identification sequence;
   (B) optionally, an additional information addition unit for adding, to the cluster, partial sequence information on the single biological units corresponding to the cluster in a database; and
   (C) a draft creation unit for creating a sequence information draft for the single biological units by using the partial sequence information of sequence information on the single biological units and sequence information on the single biological units in the database.
(Item 26) The system of item 25, further comprising a database utilization unit for utilizing a database if the system comprises the (B) addition information addition unit.
(Item 27) A system for processing sequence information on a single biological unit, the system comprising:
   A) an extraction unit for extracting genes without duplication from a draft in a database;
   B) a calculation unit for calculating the number or a ratio of corresponding drafts for each of the genes; and
   C) a selection unit for selecting a gene with the number or ratio of corresponding drafts greater than or equal to a predetermined value as a candidate of an organism lineage identification sequence.
(Item 27A) A system for processing sequence information on a single biological unit, the system comprising:
   (A) a clustering unit for clustering partial sequence information of sequence information on a plurality of single biological units for each of the same lineages based on an organism lineage identification sequence; and
   (B) an identification unit for comparing partial sequence information corresponding to the cluster in a database with partial sequence information of the cluster, calculating a degree of similarity for each partial sequence, and identifying a partial sequence with a degree of similarity greater than or equal to a predetermined degree of similarity as an organism lineage identification sequence.
(Item 28) A system for processing sequence information on a single biological unit, the system comprising:
   (D) a ranking unit for ranking partial sequence information of sequence information on a plurality of single biological units from a highest quality based on a predetermined judgment criterion;
   (E) a draft construction unit for selecting a population of a predetermined number of pieces of partial sequence information of sequence information on the plurality of single biological units from a highest ranking based on the ranking to construct a draft with a greater length than the partial sequence information from the partial sequence information; and
   (E') a selection unit for selecting a population of a set of a different number of pieces of partial sequence information of sequence information on the single biological units from the population, constructing a draft with a greater length than the partial sequence information from the partial sequence information, and selecting a draft created up to this point based on a predetermined judgment criterion.
(Item 29) The system for processing sequence information on a single biological unit of item 28, the system comprising:
   (F) a selection unit for comparing the selected draft with partial sequence information of sequence information on a single biological unit that was not selected in (E) or (E') and selecting partial sequence information of sequence information on the single biological unit having a sequence of a portion that is not included in the draft;
   (G) a draft improvement unit for creating a longer draft by using the sequence information selected in (F) and the selected draft;
   (G') optionally, a draft construction unit for repeating draft creation in (G) until the longer draft reaches a full length of sequence information; and
   (G") optionally, means for repeating the ranking, draft construction, and selection in (D), (E), and (E') of item 28 based on a judgment criterion with a lower criterion in the entire partial sequence information constituting the draft.
(Item 30) A system for processing sequence information on a single biological unit, the system comprising:
   (A) a clustering unit for clustering partial sequence information of sequence information on a plurality of single biological units for each of the same lineages based on an organism lineage identification sequence;
   (H) a reclustering unit for evaluating partial sequence information of sequence information on the plurality of single biological units constituting sequence information on a single biological unit based on an organism lineage identification sequence within the cluster of the same lineage, and reclustering within the cluster of the same lineage;
   (H') a comparison unit for comparing a sequence information draft created from the cluster of the same lineage with a sequence information draft created from the reclustered cluster; and
   (I) a registration unit for determining whether reclustering in (H) is appropriate for a result of comparison based on a predetermined judgment criterion, and, if appropriate, registering the draft in a database as a new group.
(Item 31) The system of item 30, wherein the reclustering unit performs reclustering through network analysis and community detection.
(Item 32) A system for processing sequence information on a single biological unit, the system comprising:
   (A) a clustering unit for clustering partial sequence information of sequence information on a plurality of single biological units for each of the same lineages based on an organism lineage identification sequence;
   (D) a ranking unit for ranking partial sequence information of sequence information on the plurality of single biological units belonging to the cluster of the same lineage from a highest quality based on a predetermined judgment criterion;
   (E) a draft construction unit for selecting a population of a predetermined number of pieces of partial sequence information of sequence information on the plurality of single biological units from a highest ranking based on the ranking to construct a draft with a greater length than the partial sequence information from the partial sequence information,
   (E") selecting a population of a set of a different number of pieces of partial sequence information of sequence information on the single biological units from the population, constructing a draft with a greater length than the partial sequence information from the partial sequence information, and evaluating a draft created up to this point based on a predetermined judgment criterion;
   (H) a reclustering unit for evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change due to an increase in the number in a population of a set of sequence information;
   (H') a comparison unit for comparing a sequence information draft created from the cluster of the same lineage with a sequence information draft created from the reclustered cluster; and
   (J) means for determining whether reclustering in (H) is appropriate for a result of comparison based on a predetermined judgment criterion, and, if appropriate, repeating (D) to (E') for partial sequence information of sequence information on the plurality of single biological units belonging to the reclustered cluster.
(Item A1) A method of giving an instruction to a computer to execute processing of sequence information on a single biological unit, the computer given the instruction executing:
   (A) a step of clustering partial sequence information of sequence information on a plurality of single biological units for each of the same lineages based on an organism lineage identification sequence; and
   (C) a step of creating a sequence information draft for the single biological unit by using the partial sequence information of sequence information on the single biological unit and sequence information on the single biological unit in a database created independently from the clustering.
(Item A2) The method of the preceding item, further comprising:
   (B) a step of adding, to the cluster, partial sequence information on the single biological units corresponding to the cluster in the database.
(Item A3) The method of any one of the preceding items, wherein
   (C) comprises removing a certain amount of partial sequence information comprising a sequence site found to have a large number of duplications to correct a bias in sequence reads.
(Item A4) A method of giving an instruction to a computer to execute screening of candidates of an organism lineage identification sequence, the computer given the instruction executing:
   A) a step of extracting genes without duplication from a draft in a database;
   B) a step of calculating the number or a ratio of single copy genes for each of the genes; and
   C) a step of selecting a gene with the number or ratio of single copy genes greater than or equal to a predetermined value as a candidate of an organism lineage identification sequence.
(Item A5) A method of giving an instruction to a computer to execute processing of sequence information on a single biological unit, the computer given the instruction executing:
   (D) a step of ranking partial sequence information of sequence information on the plurality of single biological units from a highest quality based on a predetermined judgment criterion;
   (E) a step of selecting a population of a predetermined number of pieces of partial sequence information of sequence information on the plurality of single biological units from a highest ranking based on the ranking to construct a draft with a greater length than the partial sequence information from the partial sequence information; and
   (E') a step of selecting a population of a set of a different number of pieces of partial sequence information of sequence information on the single biological units from the population, constructing a draft with a greater length than the partial sequence information from the partial sequence information, and selecting a draft created up to this point based on a predetermined judgment criterion.
(Item A6) The method of giving an instruction to a computer to execute processing of sequence information on a single biological unit of any one of the preceding items, the computer given the instruction executing:
   (F) a step of comparing the selected draft with partial sequence information of sequence information on a single biological unit that was not selected in (E) or (E') and selecting partial sequence information of sequence information on the single biological unit having a sequence of a portion that is not included in the draft;
   (G) a step of creating a longer draft by using the sequence information selected in (F) and the selected draft;
   (G') optionally, a step of repeating (G) until the longer draft reaches a full length of sequence information; and
   (G") optionally, a step of repeating the steps of item 5 based on a judgment criterion with a lower criterion in the entire partial sequence information constituting the draft.
(Item A7) A method of giving an instruction to a computer to execute processing of sequence information on a single biological unit, the computer given the instruction executing:
   (A) a step of clustering partial sequence information of sequence information on a plurality of single biological units for each of the same lineages based on an organism lineage identification sequence;
   (H) a step of evaluating partial sequence information of sequence information on the plurality of single biological units constituting sequence information on a single biological unit based on an organism lineage identification sequence within the cluster of the same lineage, and reclustering within the cluster of the same lineage;
   (H') a step of comparing a sequence information draft created from the cluster of the same lineage with a sequence information draft created from the reclustered cluster; and
   (I) a step of determining whether reclustering in (H) is appropriate for a result of comparison based on a predetermined judgment criterion, and, if appropriate, registering the draft in a database as a new group.
(Item A8) The method of any one of the preceding items, wherein the reclustering is performed through network analysis and community detection.
(Item A9) A method of giving an instruction to a computer to execute processing of sequence information on a single biological unit, the computer given the instruction executing:
   (A) a step of clustering partial sequence information of sequence information on a plurality of single biological units for each of the same lineages based on an organism lineage identification sequence;
   (D) a step of ranking partial sequence information of sequence information on the plurality of single biological units belonging to the cluster of the same lineage from a highest quality based on a predetermined judgment criterion;
   (E) a step of selecting a population of a predetermined number of pieces of partial sequence information of sequence information on the plurality of single biological units from a highest ranking based on the ranking to construct a draft with a greater length than the partial sequence information from the partial sequence information;
   (E") a step of selecting a population of a set of a different number of pieces of partial sequence information of sequence information on the single biological units from the population, constructing a draft with a greater length than the partial sequence information from the partial sequence information, and evaluating a draft created up to this point based on a predetermined judgment criterion;
   (H) a step of evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change due to an increase in the number in a population of a set of sequence information;
   (H') a step of comparing a sequence information draft created from the cluster of the same lineage with a sequence information draft created from the reclustered cluster; and (J) a step of determining whether reclustering in (H) is appropriate for a result of comparison based on a predetermined judgment criterion, and, if appropriate, repeating (D) to (E') for partial sequence information of sequence information on the plurality of single biological units belonging to the reclustered cluster.
(Item A10) The method of any one of the preceding items, wherein the partial sequence information is determined by long-read sequencing.
(Item A11) A program for implementing a method of processing sequence information on a single biological unit on a computer, the method comprising:
   (A) a step of clustering partial sequence information of sequence information on a plurality of single biological units for each of the same lineages based on an organism lineage identification sequence; and
   (C) a step of creating a sequence information draft for the single biological units by using the partial sequence information of sequence information on the single biological units and sequence information on the single biological units in a database created independently from the clustering.
(Item A12) The program of the preceding item, further comprising:
   (B) a step of adding, to the cluster, partial sequence information on the single biological units corresponding to the cluster in a database.
(Item A13) The program of any one of the preceding items, wherein (C) comprises removing a certain amount of partial sequence information comprising a sequence site found to have a large number of duplications to correct a bias in sequence reads.
(Item A14) A program for implementing a method of screening candidates of an organism lineage identification sequence on a computer, the method comprising:
   A) a step of extracting genes without duplication from a draft in a database;
   B) a step of calculating the number or a ratio of single copy genes for each of the genes; and
   C) a step of selecting a gene with the number or ratio of single copy genes greater than or equal to a predetermined value as a candidate of an organism lineage identification sequence.
(Item A15) A program for implementing a method of processing sequence information on a single biological unit on a computer, the method comprising:
   (D) a step of ranking partial sequence information of sequence information on the plurality of single biological units from a highest quality based on a predetermined judgment criterion;
   (E) a step of selecting a population of a predetermined number of pieces of partial sequence information of sequence information on the plurality of single biological units from a highest ranking based on the ranking to construct a draft with a greater length than the partial sequence information from the partial sequence information; and
   (E') a step of selecting a population of a set of a different number of pieces of partial sequence information of sequence information on the single biological units from the population, constructing a draft with a greater length than the partial sequence information from the partial sequence information, and selecting a draft created up to this point based on a predetermined judgment criterion.
(Item A16) The program for implementing a method of processing sequence information on a single biological unit on a computer of any one of the preceding items, the method comprising:
   (F) a step of comparing the selected draft with partial sequence information of sequence information on a single biological unit that was not selected in (E) or (E') and selecting partial sequence information of sequence information on the single biological unit having a sequence of a portion that is not included in the draft;
   (G) a step of creating a longer draft by using the sequence information selected in (F) and the selected draft;
   (G') optionally, a step of repeating (G) until the longer draft reaches a full length of sequence information; and
   (G") optionally, a step of repeating the steps of item 15 based on a judgment criterion with a lower criterion in the entire partial sequence information constituting the draft.
(Item A17) A program for implementing a method of processing sequence information on a single biological unit on a computer, the method comprising:
   (A) a step of clustering partial sequence information of sequence information on a plurality of single biological units for each of the same lineages based on an organism lineage identification sequence;
   (H) a step of evaluating partial sequence information of sequence information on the plurality of single biological units constituting sequence information on a single biological unit based on an organism lineage identification sequence within the cluster of the same lineage, and reclustering within the cluster of the same lineage;
   (H') a step of comparing a sequence information draft created from the cluster of the same lineage with a sequence information draft created from the reclustered cluster; and (I) a step of determining whether reclustering in (H) is appropriate for a result of comparison based on a predetermined judgment criterion, and, if appropriate, registering the draft in a database as a new group.
(Item A18) The program of any one of the preceding items, wherein the reclustering is performed through network analysis and community detection.
(Item A19) A program for implementing a method of processing sequence information on a single biological unit on a computer, the method comprising:
   (A) a step of clustering partial sequence information of sequence information on a plurality of single biological units for each of the same lineages based on an organism lineage identification sequence;
   (D) a step of ranking partial sequence information of sequence information on the plurality of single biological units belonging to the cluster of the same lineage from a highest quality based on a predetermined judgment criterion;
   (E) a step of selecting a population of a predetermined number of pieces of partial sequence information of sequence information on the plurality of single biological units from a highest ranking based on the ranking to construct a draft with a greater length than the partial sequence information from the partial sequence information;
   (E") a step of selecting a population of a set of a different number of pieces of partial sequence information of sequence information on the single biological units from the population, constructing a draft with a greater length than the partial sequence information from the partial sequence information, and evaluating a draft created up to this point based on a predetermined judgment criterion;
   (H) a step of evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change due to an increase in the number in a population of a set of sequence information;
   (H') a step of comparing a sequence information draft created from the cluster of the same lineage with a sequence information draft created from the reclustered cluster; and (J) a step of determining whether reclustering in (H) is appropriate for a result of comparison based on a predetermined judgment criterion, and, if appropriate, repeating (D) to (E') for partial sequence information of sequence information on the plurality of single biological units belonging to the reclustered cluster.
(Item A20) The program of any one of the preceding items, wherein the partial sequence information is determined by long-read sequencing.
(Item A21) A recording medium storing a program for implementing a method of processing sequence information on a single biological unit on a computer, the method comprising:
   (A) a step of clustering partial sequence information of sequence information on a plurality of single biological units for each of the same lineages based on an organism lineage identification sequence; and
   (C) a step of creating a sequence information draft for the single biological units by using the partial sequence information of sequence information on the single biological units and sequence information on the single biological units in a database created independently from the clustering.
(Item A22) The recording medium of the preceding item, further comprising:
   (B) a step of adding, to the cluster, partial sequence information on the single biological units corresponding to the cluster in the database.
(Item A23) The recording medium of any one of the preceding items, wherein (C) comprises removing a certain amount of partial sequence information comprising a sequence site found to have a large number of duplications to correct a bias in sequence reads.
(Item A24) A recording medium storing a program for implementing a method of screening candidates of an organism lineage identification sequence on a computer, the method comprising:
   A) a step of extracting genes without duplication from a draft in a database;
   B) a step of calculating the number or a ratio of single copy genes for each of the genes; and
   C) a step of selecting a gene with the number or ratio of single copy genes greater than or equal to a predetermined value as a candidate of an organism lineage identification sequence.
(Item A25) A recording medium storing a program for implementing a method of processing sequence information on a single biological unit on a computer, the method comprising:
   (D) a step of ranking partial sequence information of sequence information on a plurality of single biological units from a highest quality based on a predetermined judgment criterion;
   (E) a step of selecting a population of a predetermined number of pieces of partial sequence information of sequence information on the plurality of single biological units from a highest ranking based on the ranking to construct a draft with a greater length than the partial sequence information from the partial sequence information; and
   (E') a step of selecting a population of a set of a different number of pieces of partial sequence information of sequence information on the single biological units from the population, constructing a draft with a greater length than the partial sequence information from the partial sequence information, and selecting a draft created up to this point based on a predetermined judgment criterion.
(Item A26) The recording medium storing a program for implementing a method of processing sequence information on a single biological unit on a computer of any one of the preceding items, the method comprising:
   (F) a step of comparing the selected draft with partial sequence information of sequence information on a single biological unit that was not selected in (E) or (E') and selecting partial sequence information of sequence information on the single biological unit having a sequence of a portion that is not included in the draft;
   (G) a step of creating a longer draft by using the sequence information selected in (F) and the selected draft;
   (G') optionally, a step of repeating (G) until the longer draft reaches a full length of sequence information; and
   (G") optionally, a step of repeating the steps of item 25 based on a judgment criterion with a lower criterion in the entire partial sequence information constituting the draft.
(Item A27) A recording medium storing a program for implementing a method of processing sequence information on a single biological unit on a computer, the method comprising:
   (A) a step of clustering partial sequence information of sequence information on a plurality of single biological units for each of the same lineages based on an organism lineage identification sequence;
   (H) a step of evaluating partial sequence information of sequence information on the plurality of single biological units constituting sequence information on a single biological unit based on an organism lineage identification sequence within the cluster of the same lineage, and reclustering within the cluster of the same lineage;
   (H') a step of comparing a sequence information draft created from the cluster of the same lineage with a sequence information draft created from the reclustered cluster; and (I) a step of determining whether reclustering in (H) is appropriate for a result of comparison based on a predetermined judgment criterion, and, if appropriate, registering the draft in a database as a new group.
(Item A28) The recording medium of any one of the preceding items, wherein the reclustering is performed through network analysis and community detection.
(Item A29) A recording medium storing a program for implementing a method of processing sequence information on a single biological unit on a computer, the method comprising:
   (A) a step of clustering partial sequence information of sequence information on a plurality of single biological units for each of the same lineages based on an organism lineage identification sequence;
   (D) a step of ranking partial sequence information of sequence information on the plurality of single biological units belonging to the cluster of the same lineage from a highest quality based on a predetermined judgment criterion;
   (E) a step of selecting a population of a predetermined number of pieces of partial sequence information of sequence information on the plurality of single biological units from a highest ranking based on the ranking to construct a draft with a greater length than the partial sequence information from the partial sequence information;
   (E") a step of selecting a population of a set of a different number of pieces of partial sequence information of sequence information on the single biological unit from the population, constructing a draft with a greater length than the partial sequence information from the partial sequence information, and evaluating a draft created up to this point based on a predetermined judgment criterion;
   (H) a step of evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change due to an increase in the number in a population of a set of sequence information;
   (H') a step of comparing a sequence information draft created from the cluster of the same lineage with a sequence information draft created from the reclustered cluster; and (J) a step of determining whether reclustering in (H) is appropriate for a result of comparison based on a predetermined judgment criterion, and, if appropriate, repeating (D) to (E') for partial sequence information of sequence information on the plurality of single biological units belonging to the reclustered cluster.
(Item A30) The recording medium of any one of the preceding items, wherein the partial sequence information is determined by long-read sequencing.
(Item A31) A system for processing sequence information on a single biological unit, the system comprising:
   (A) a clustering unit for clustering partial sequence information of sequence information on a plurality of single biological units for each of the same lineages based on an organism lineage identification sequence; and
   (C) a draft creation unit for creating a sequence information draft for the single biological units by using the partial sequence information of sequence information on the single biological units and sequence information on the single biological units in a database created independently from clustering by the clustering unit of (A).
(Item A32) The system of the preceding item, further comprising:
   (B) an additional information addition unit for adding, to the cluster, partial sequence information on the single biological units corresponding to the cluster in the database.
(Item A33) The system of any one of the preceding items, wherein (C) comprises removing a certain amount of partial sequence information comprising a sequence site found to have a large number of duplications to correct a bias in sequence reads.
(Item A34) A system for screening candidates of an organism lineage identification sequence, the system comprising:
   A) an extraction unit for extracting genes without duplication from a draft in a database;
   B) a calculation unit for calculating the number or a ratio of single copy genes for each of the genes; and
   C) a selection unit for selecting a gene with the number or ratio of single copy genes greater than or equal to a predetermined value as a candidate of an organism lineage identification sequence.
(Item A35) A system for processing sequence information on a single biological unit, the system comprising:
   (D) a ranking unit for ranking partial sequence information of sequence information on a plurality of single biological units from a highest quality based on a predetermined judgment criterion;
   (E) a draft construction unit for selecting a population of a predetermined number of pieces of partial sequence information of sequence information on the plurality of single biological units from a highest ranking based on the ranking to construct a draft with a greater length than the partial sequence information from the partial sequence information; and
   (E') a selection unit for selecting a population of a set of a different number of pieces of partial sequence information of sequence information on the single biological units from the population, constructing a draft with a greater length than the partial sequence information from the partial sequence information, and selecting a draft created up to this point based on a predetermined judgment criterion.
(Item A36) The system for processing sequence information on a single biological unit of any one of the preceding items, the system comprising:
   (F) a selection unit for comparing the selected draft with partial sequence information of sequence information on a single biological unit that was not selected in (E) or (E') and selecting partial sequence information of sequence information on the single biological unit having a sequence of a portion that is not included in the draft;
   (G) a draft improvement unit for creating a longer draft by using the sequence information selected in (F) and the selected draft;
   (G') optionally, a draft construction unit for repeating draft creation in (G) until the longer draft reaches a full length of sequence information; and
   (G") optionally, means for repeating the ranking, draft construction, and selection of (D), (E), and (E') of item 35 based on a judgment criterion with a lower criterion in the entire partial sequence information constituting the draft.
(Item A37) A system for processing sequence information on a single biological unit, the system comprising:
   (A) a clustering unit for clustering partial sequence information of sequence information on a plurality of single biological units for each of the same lineages based on an organism lineage identification sequence;
   (H) a reclustering unit for evaluating partial sequence information of sequence information on the plurality of single biological units constituting sequence information on a single biological unit based on an organism lineage identification sequence within the cluster of the same lineage, and reclustering within the cluster of the same lineage;
   (H') a comparison unit for comparing a sequence information draft created from the cluster of the same lineage with a sequence information draft created from the reclustered cluster; and
   (I) a registration unit for determining whether reclustering in (H) is appropriate for a result of comparison based on a predetermined judgment criterion, and, if appropriate, registering the draft in a database as a new group.
(Item A38) The system of any one of the preceding items, wherein the reclustering unit performs reclustering through network analysis and community detection.
(Item A39) A system for processing sequence information on a single biological unit, the system comprising:
   (A) a clustering unit for clustering partial sequence information of sequence information on a plurality of single biological units for each of the same lineages based on an organism lineage identification sequence;
   (D) a ranking unit for ranking partial sequence information of sequence information on the plurality of single biological units belonging to the cluster of the same lineage from a highest quality based on a predetermined judgment criterion;
   (E) a draft construction unit for selecting a population of a predetermined number of pieces of partial sequence information of sequence information on the plurality of single biological units from a highest ranking based on the ranking to construct a draft with a greater length than the partial sequence information from the partial sequence information,
   (E") selecting a population of a set of a different number of pieces of partial sequence information of sequence information on the single biological units from the population, constructing a draft with a greater length than the partial sequence information from the partial sequence information, and evaluating a draft created up to this point based on a predetermined judgment criterion;
   (H) a reclustering unit for evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change due to an increase in the number in a population of a set of sequence information;
   (H') a comparison unit for comparing a sequence information draft created from the cluster of the same lineage with a sequence information draft created from the reclustered cluster; and
   (J) means for determining whether reclustering in (H) is appropriate for a result of comparison based on a predetermined judgment criterion, and, if appropriate, repeating (D) to (E') for partial sequence information of sequence information on the plurality of single biological units belonging to the reclustered cluster.
(Item A40) The system of any one of the preceding items, wherein the partial sequence information is determined by long-read sequencing.
(Item B1) A data structure containing partial sequence information of sequence information on a plurality of single biological units clustered for each of the same lineages based on an organism lineage identification sequence.
(Item B2) The data structure of any one of the preceding items, wherein the partial sequence information contained in the data structure is derived from two or more independently clustered and created databases.
(Item B3) The data structure of any one of the preceding items, wherein information associated with the independently performed clustering is linked to and stored with the partial sequence information.
(Item B4) The data structure of any one of the preceding items, wherein the partial sequence information, as a whole, constitutes genomic information.
(Item B5) The data structure of any one of the preceding items, wherein the partial sequence information is collected for each single biological unit.
(Item B6) The data structure of any one of the preceding items, wherein the partial sequence information is linked to and stored with identification information (ID information) on a single biological unit from which the partial sequence information is derived.
(Item B7) A data structure for a single biological unit from integrating a plurality of data structures, containing partial sequence information of sequence information on a plurality of single biological units clustered for each of the same lineages based on an organism lineage identification sequence.
(Item B8) The data structure of item B7, further comprising one or more features of any one or more of the preceding items.

The present disclosure is intended so that one or more of the features described above can be provided not only as the explicitly disclosed combinations, but also as other combinations thereof. Additional embodiments and advantages of the present disclosure are recognized by those skilled in the art by reading and understanding the following detailed description as needed.

### [Advantageous Effects of Invention]

With the present disclosure, sequence information on a single biological unit at a single biological unit level can be provided more accurately. Use of the present disclosure enables elucidation of a nearly complete genome sequence of microorganisms that cannot be cultured and analysis of genetic heterogeneity between microorganisms of the same strain.

### [Brief Description of Drawings]

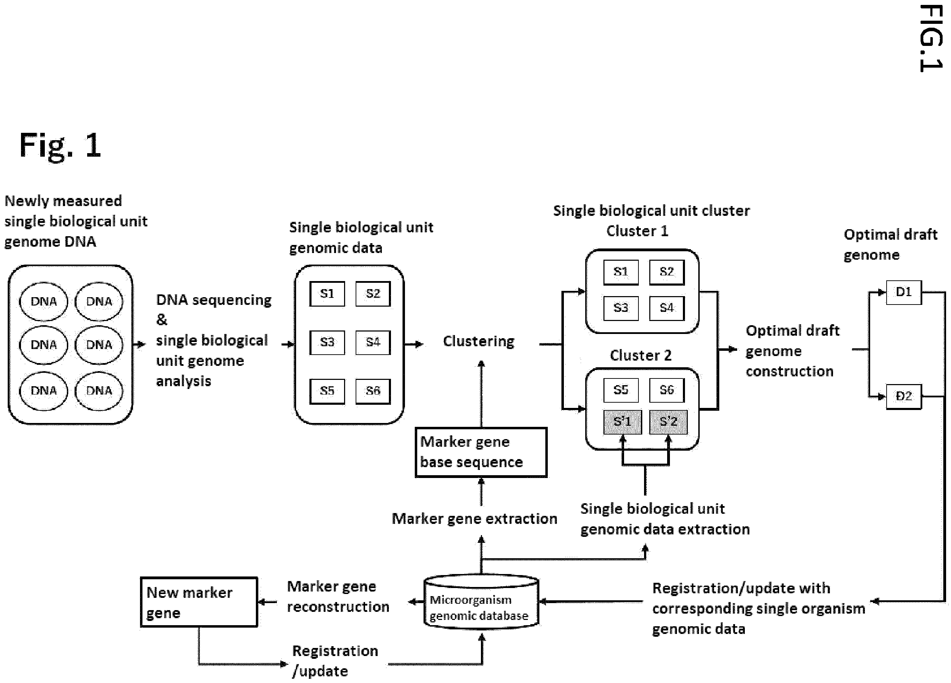

[Figure 1] Figure **1** is an overall schematic diagram of the present disclosure. The present disclosure clusters partial sequence information on a single biological unit that is newly acquired for the same lineage by using an organism lineage identification sequence registered in the present system, and constructs, combines clustered partial sequence information on a plurality of single biological units to construct the optimal draft genomic sequence. A draft genomic sequence registered in a microorganism genome database is updated upon each measurement/registration of a single biological unit that is new to gradually improve the quality.

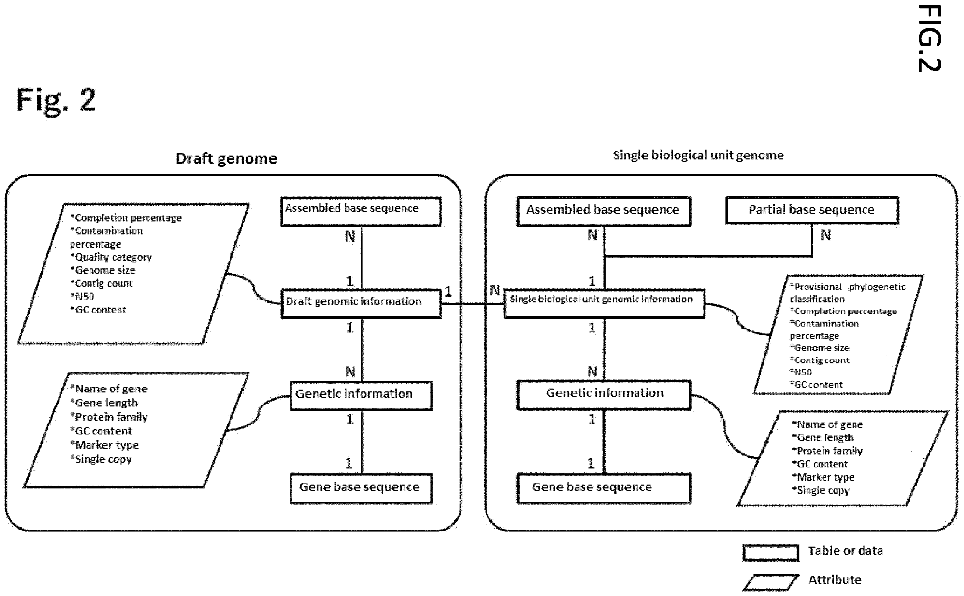

[Figure 2] Figure **2** is a microorganism database structure used in the present system. A microorganism genome database is constructed with single biological unit genomic information and draft genomic information created by integration therewith. Data associated with genome sequences such as provisional phylogenetic classification, completion percentage, contamination percentage, quality category, contig count, N50 statistical value, and GC content is recorded in the draft genomic information. A plurality of assembled base sequences and genetic information are associated with one piece of draft genomic information. Data associated with genes such as the name of gene, gene length, protein family, GC content, marker type, and single copy is recorded in the genetic information. One gene base sequence is associated with one piece of genetic information. Single biological unit genomic information is also associated with similar data as the draft genomic information. A plurality of assembled base sequences and genetic information are associated with one piece of single biological unit genomic information in the same manner as the draft genomic information, and one gene base sequence is associated with one piece of genetic information. A plurality of partial base sequences are associated with one piece of single biological unit genomic information.

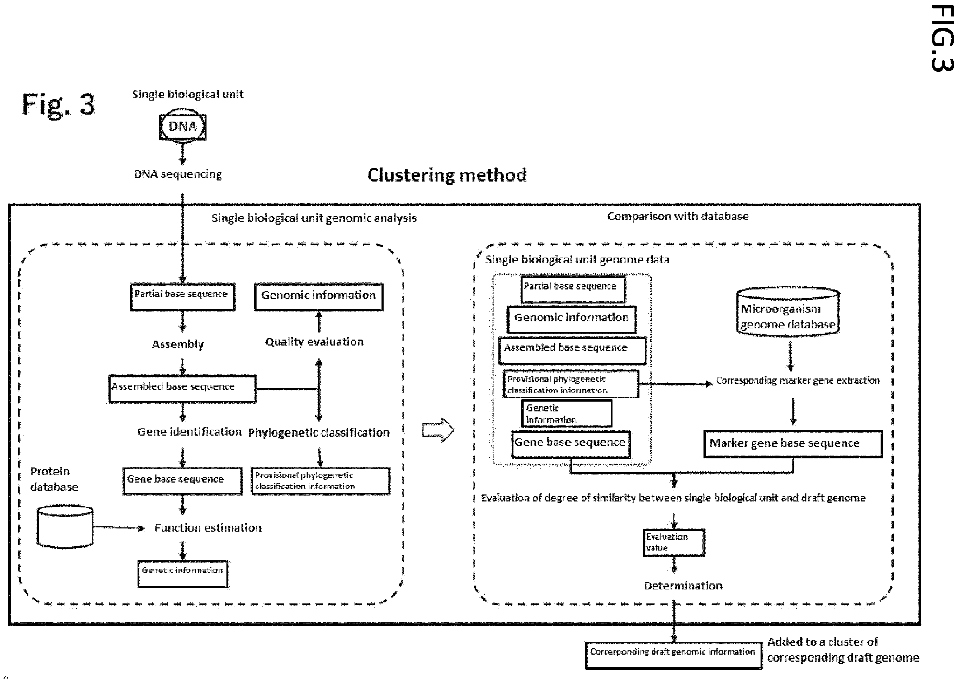

[Figure 3] Figure **3** is a method of clustering single biological units newly measured from a microorganism database for the same lineage. The genomic DNA of a single biological unit is measured with a DNA sequencer to obtain a partial base sequence. Partial base sequence is assembled to obtain an assembled base sequence. Gene identification is performed on the assembled base sequence to obtain a gene base sequence. The function is estimated using a protein database or the like for each gene base sequence to obtain genetic information. At the same time, the assembled base sequence is phylogenetically classified to obtain provisional phylogenetic classification information. The quality of the assembled base sequence is also evaluated to obtain genomic information. A partial base sequence, genomic information, assembled base sequence, phylogenetic classification information, genetic information, and gene base sequence obtained from analyzing a single biological unit are used as single biological unit genomic data. Draft genomic information of the same lineage is searched using phylogenetic classification information from a microorganism genome database. Furthermore, an organism lineage identification sequence and a protein family thereof are obtained based on genetic information corresponding to the draft genomic information. Genetic information and gene base sequence for the same protein family as the obtained protein family are extracted from single biological unit genomic data. The identity is calculated with the combination of an organism lineage identification sequence of a draft genome and a corresponding gene base sequence of a single biological unit genome. Combinations with identity of a certain level or less are excluded from subsequent processing. A gene with the highest identify for the organism lineage identification sequence is identified as an organism lineage identification sequence of a single biological unit. The degree of similarity of a draft genome to a single biological unit is evaluated by the weighted average of matched base sequence lengths and homology, etc. The draft genome with the highest evaluation is considered as the cluster to which the single biological unit belongs. If there are multiple draft genomes with the same evaluation value, the same evaluation is performed using the entire assembled base sequence instead of an organism lineage identification sequence for determination.

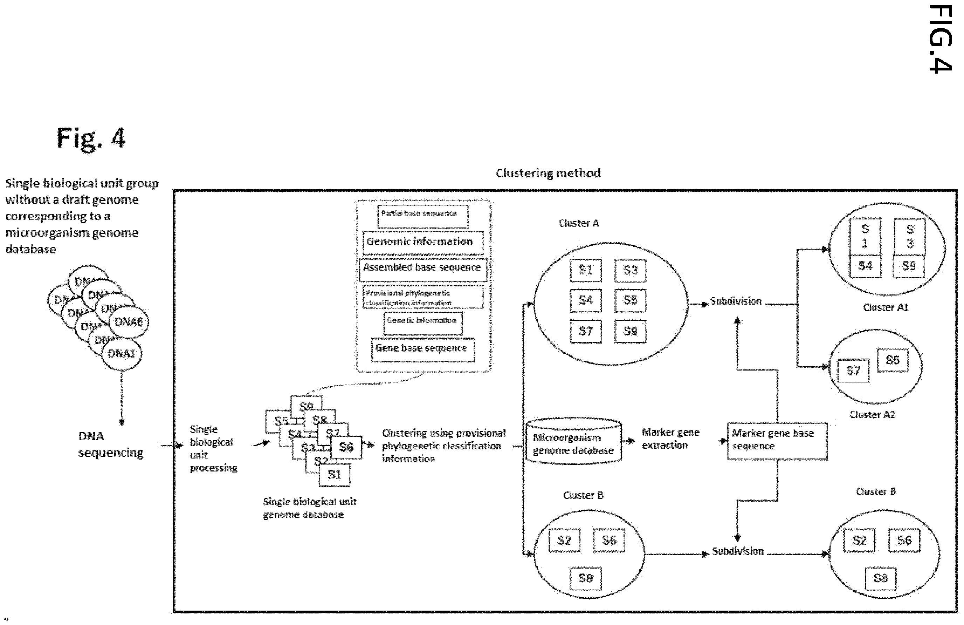

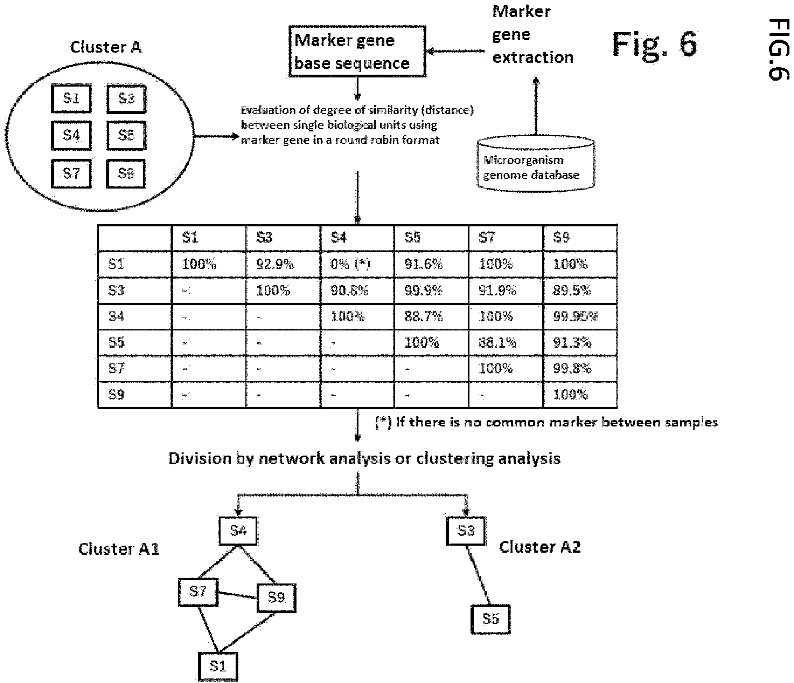

[Figure 4] Figure **4** is a method of clustering newly measured single biological units for the same lineages without using a microorganism database. A provisional cluster is constructed by provisional phylogenetic classification for single biological unit genomic data without a corresponding draft genome in a microorganism genome database. It is determined whether single biological unit genomic data belonging to each provisional cluster should be further divided by using an organism lineage identification sequence in a microorganism genome database. A division method is shown in Figure **6****.**

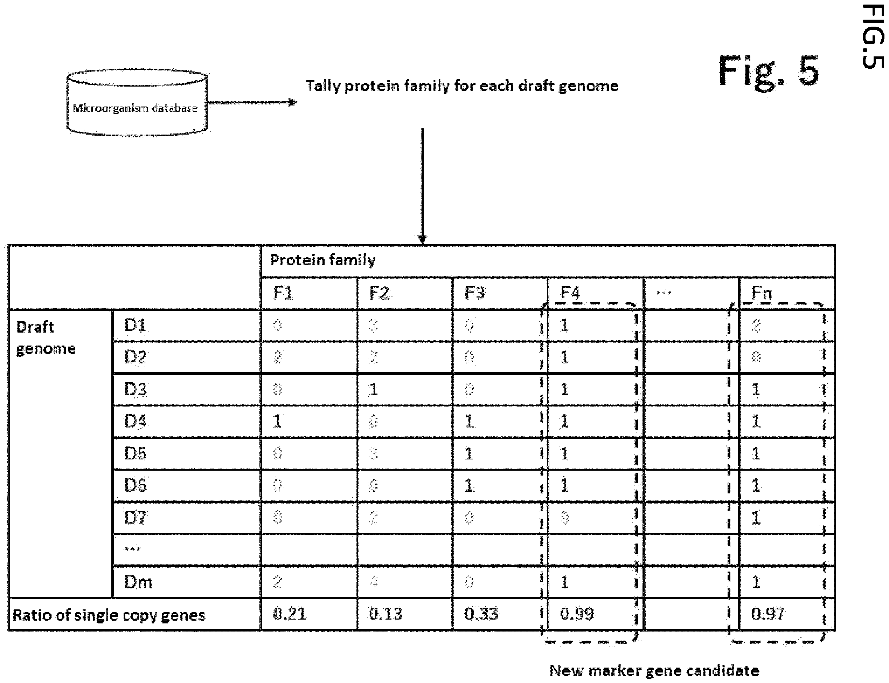

[Figure 5] Figure **5** is a method of newly identifying an organism lineage identification sequence by using genetic information registered in a microorganism database. In a microorganism genome database, the quality of draft genomic information and corresponding genetic information gradually improves as data is accumulated. In this regard, a method of re-identifying a new organism lineage identification sequence from high quality draft genomic information and genetic information is implemented in the present system. A corresponding protein family is extracted for each registered draft genome from a microorganism genome database, and the frequency thereof is calculated to create a frequency matrix. At this time, low quality draft genomes can be excluded. A protein family represented at a plurality of frequencies for one draft genome is excluded in a frequency matrix due to the possibility of contamination. However, if corresponding to one protein family, the protein family is identified as a single copy gene. If there is a single copy gene of a protein family at a certain ratio or higher within the whole draft genome, a gene corresponding to the protein family is a candidate of an organism lineage identification sequence. Candidates are sorted in ascending order of highest ratio, and candidates at or above a certain criterion (e.g., 90% or greater) or several of the top ranking candidates are employed as a new organism lineage identification sequence.
[Figure 6] Figure **6** is a method of subdividing newly measured single biological units within a cluster that are considered the same lineage. The degree of similarity (distance) of each single biological unit is evaluated in a round robin format with an organism lineage identification sequence extracted from a microorganism genome database for newly measured single biological units determined to be in the same cluster. Network analysis or cluster analysis is performed using a degree of similarity (distance) matrix to subdivide single biological units.

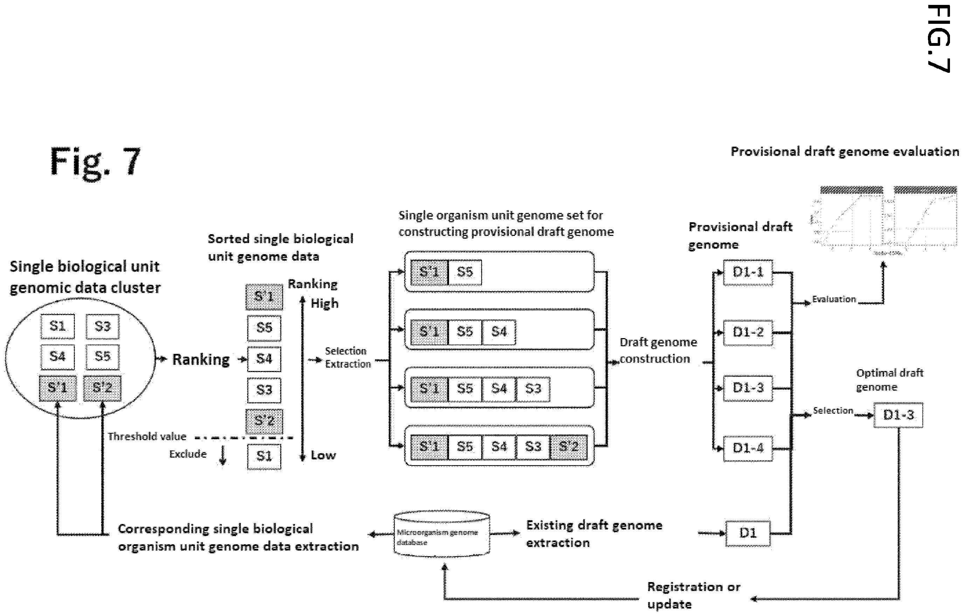

[Figure 7] Figure **7** is a method of constructing the optimal draft genome depending on a single biological unit within a cluster that is considered to be of the same lineage. New single organism unit genomic data considered to be of the same lineage, and unit genomic data for the same lineage from a microorganism genomic data if this is available, is extracted as a cluster. Single biological unit genomic data within a cluster is rearranged based on a predetermined judgment criterion (e.g., completion percentage or contamination percentage). Unit genomic data that does not reach a certain level is excluded in subsequent processing. Two highest ranking rearranged single biological unit genomic data are selected as the single biological unit genomic data set for constructing a provisional draft genome. A plurality of single biological units genomic data sets for constructing a provisional draft genome, to which single biological unit genomic data is sequentially added in the same manner from the highest ranking, are constructed. A provisional draft genome is constructed by using ccSAG on these single biological unit genomic data sets for constructing a provisional draft genome. The provisional draft genome with the highest criterion is selected as the optimal draft genome based on a predetermined judgment criterion (e.g., completion percentage or contamination percentage). If a criterion is higher than an existing draft genome in a microorganism genome database, the newly created draft genome is registered and updated in a microorganism genome database. Further, data from evaluating the provisional draft genome is created using the number of single biological unit genomic data constructing the provisional draft genome as the explanatory variable and a criterion value (e.g., completion percentage or contamination percentage) as the objective variable. This is useful when it is determined that the quality of the draft genome has converged so that further improvement cannot be expected even if single biological unit genomic data is added.

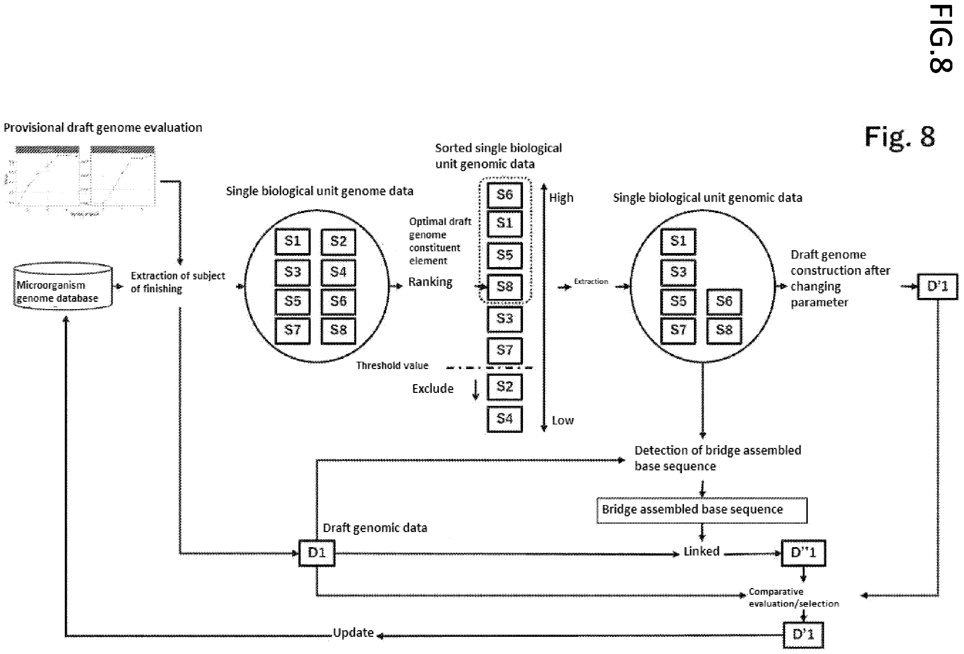

[Figure 8] Figure **8** is a method of constructing a higher quality draft genome for a draft genome registered in a microorganism database. A draft genome whose quality is estimated to have converged among draft genomes within a microorganism genome database is extracted as a subject of finishing. The extracted single biological unit genomic data is rearranged based on a predetermined judgment criterion (e.g., completion percentage or contamination percentage). Single biological unit genomic data that does not reach a certain level is excluded from subsequent processing. A draft genome is constructed by setting a parameter with a higher tolerance than normal draft genome construction with respect to the extracted single biological units. Meanwhile, homology search is performed between an assembled base sequence of single biological unit genomic data for reconstructing a draft genome and an assembled base sequence of a draft genome to detect a bridge assembled base sequence for linking the assembled base sequence of the draft genome. If a bridge assembled base sequence is able to be detected, this is used to link the assembled base sequence of the draft genome. Two sets of draft genomic data constructed in this manner and draft genomic data already registered in a microorganism genome database are compared and evaluated, and data with a higher criterion value is selected. If new draft genomic data is selected, this is registered to update the microorganism database.

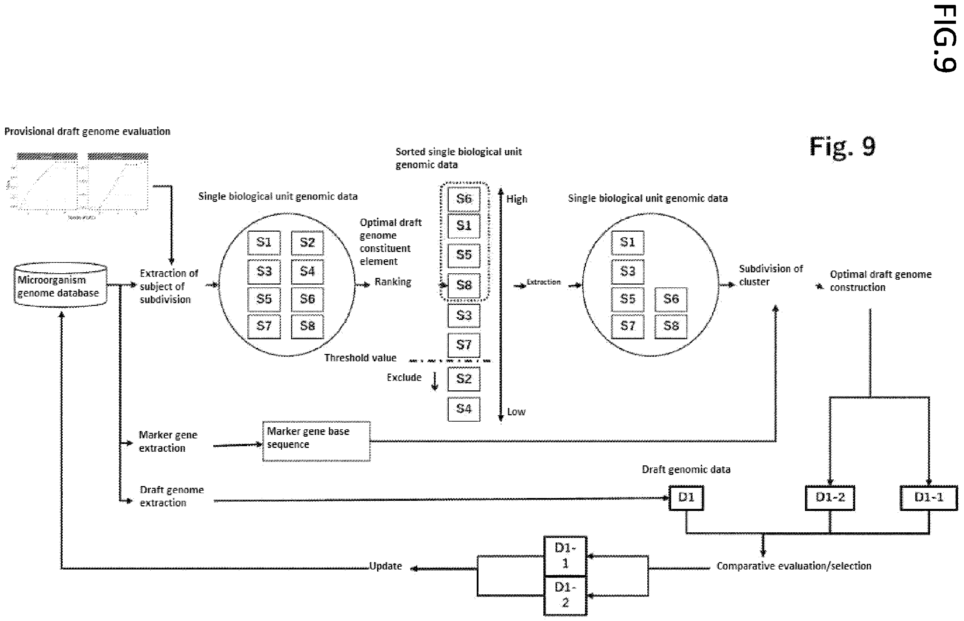

[Figure 9] Figure **9** is a method for further subdividing draft genomes registered in a microorganism database. A draft genome whose quality is estimated to have converged among draft genomes within a microorganism genome database is extracted as a subject of subdivision. The extracted single biological unit genomic data is rearranged based on a predetermined judgment criterion (e.g., completion percentage or contamination percentage). Single biological unit genomic data that does not reach a certain level is excluded from subsequent processing. The subdivision processing in Figure **6** is performed on the extracted single biological units. A draft genome is constructed for single biological unit genomic data for each subdivided cluster. Draft genomic data constructed by subdivision and draft genomic data already registered in a microorganism genome database are compared, and data with a higher criterion value is selected. If new draft genomic data is selected, this is registered to update the microorganism database.

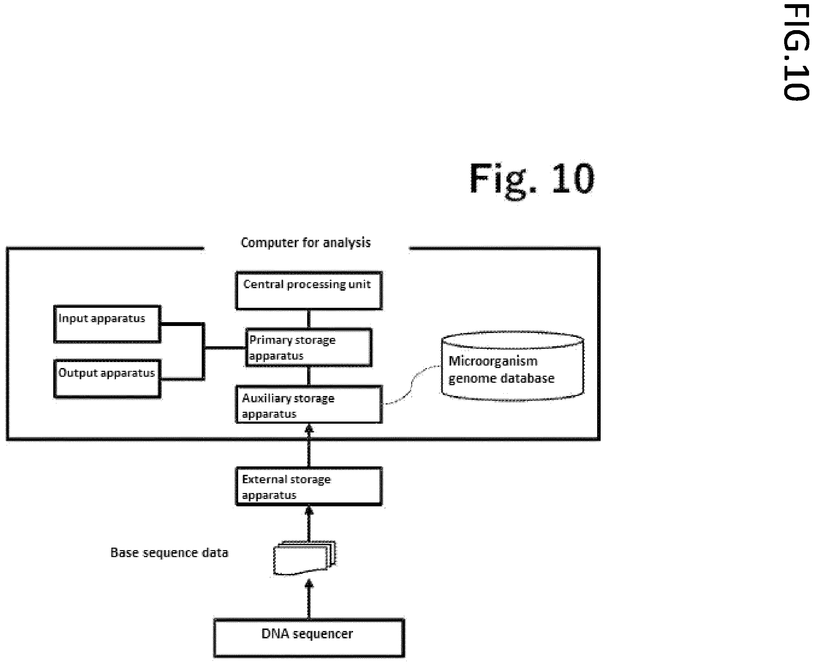

[Figure 10] Figure **10** is a diagram showing the system configuration for standalone analysis. Partial base sequence data for a single biological unit outputted from a DNA base sequence is recorded in an auxiliary storage device of a computer for analysis via an external storage device such as a portable HDD. Further, a group of programs for sequence processing and a microorganism genome database are recorded on the auxiliary storage device. A program and partial base sequence data are loaded onto the primary storage device from the auxiliary storage device, and processing is executed by a central processing unit. A series of processing is performed with an input device such as a keyboard and a mouse. Processing results are outputted to an output device such as a monitor and the auxiliary storage device.

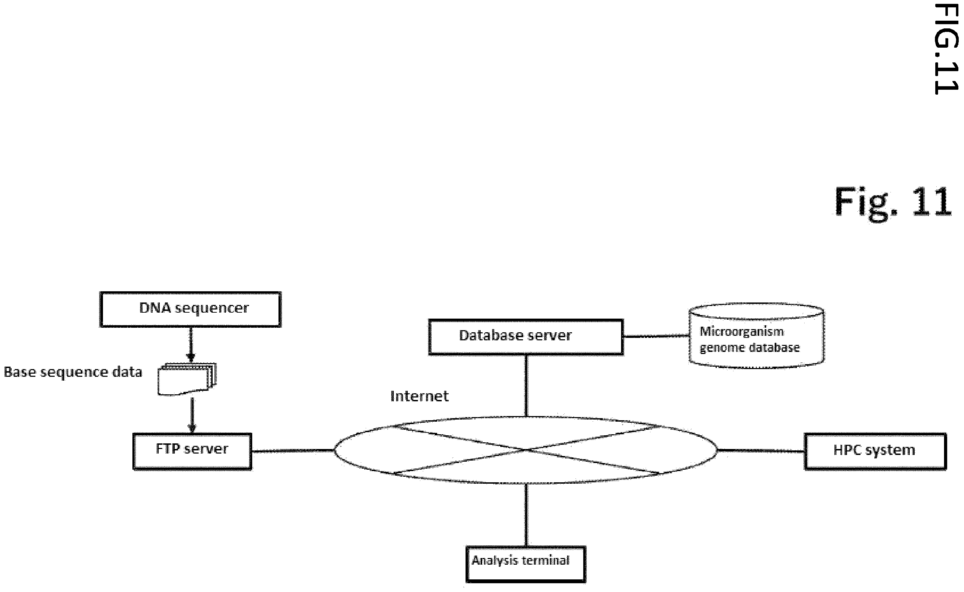

[Figure 11] Figure **11** is a diagram showing the system configuration for processing via the Internet such as cloud analysis. Partial base sequence data for a single biological unit outputted from a DNA base sequence is recorded on an FTP server or the like and can be downloaded via the Internet. The partial base sequence data is uploaded to an HPC (High-Performance Computing) system and processed on the HPC system. A microorganism genome database can be accessed via a database server, or the database itself can be downloaded onto the HPC system for use. The series of processing is performed by an analysis terminal connected to the Internet.

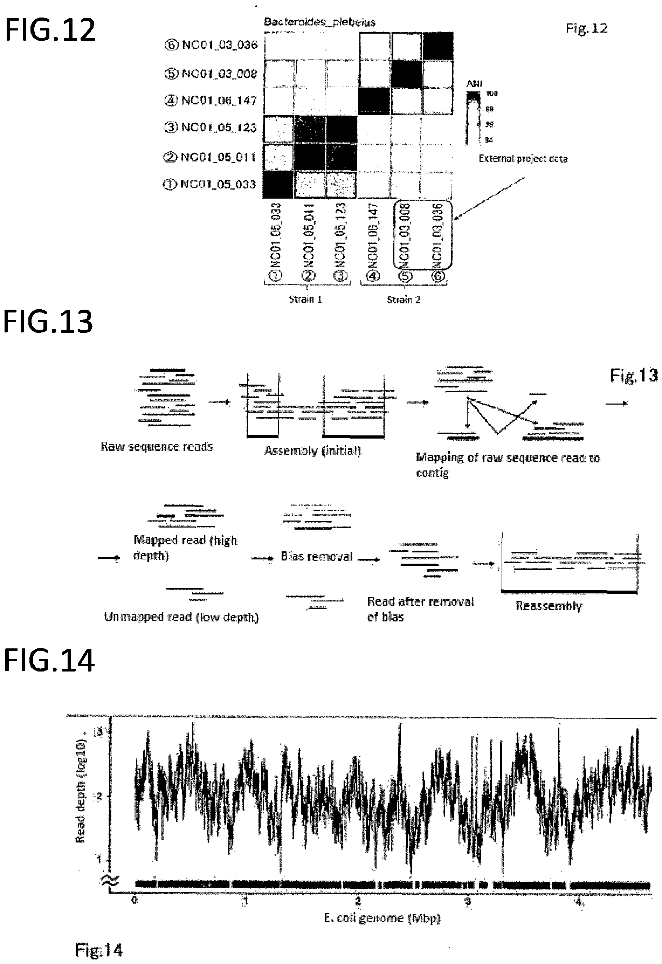

[Figure 12] Figure **12** is a diagram showing that a high quality genome sequence can be constructed by adding a sequence in an external database to a cluster.
[Figure 13] Figure **13** is a schematic diagram for bias homogenization processing.
[Figure 14] Figure **14** is a diagram showing bias evaluation of E. coli SAG sequence data prior to bias homogenization processing and the acquired genome sequence.

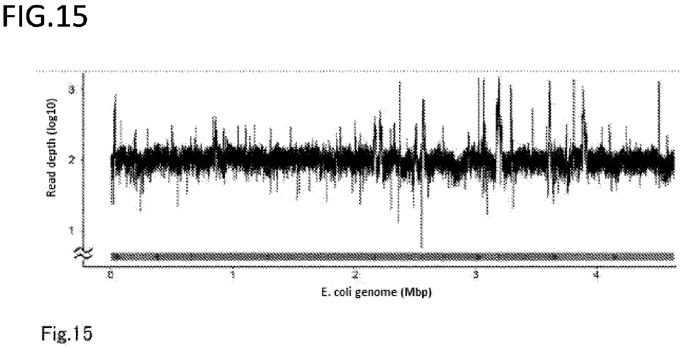

[Figure 15] Figure **15** is a diagram showing bias evaluation of E. coli SAG sequence data after bias homogenization processing and the acquired genome sequence.

### [Description of Embodiments]

The present disclosure is described hereinafter while showing the best mode thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the disclosure pertains. In case of a contradiction, the present specification (including the definitions) takes precedence.

### (Definitions, etc.)

The definitions of the terms and/or basic technical matters especially used herein are described hereinafter when appropriate.

As used herein, "single biological unit" refers to a unit with genetic information or other information on a biomolecule. A single biological unit can include cells, cell-like constructs, and the like, but is not limited thereto. A single biological unit can also include artificially produced cells (so-called artificial cells), digital cells (provided as information), and the like.

As used herein, "cell" refers to any particle that encapsulates a molecule with genetic information and can be replicated (regardless of whether the cell can be replicated independently). As used herein, "cell" includes cells of unicellular organisms, bacteria, cells derived from a multicellular organism, fungi, and the like.

As used herein, "cell-like construct" refers to any particle that encapsulates a molecule with genetic information. As used herein, "cell-like construct" includes intracellular organelles such as mitochondria, cell nucleus, and chloroplast, viruses, and the like.

As used herein, "genetic information or other information on a biomolecule" refers to information specifying a biomolecule or an analog thereof. Genetic information or other information on a biomolecule can include structural information on a nucleic acid, amino acid, lipid, or sugar chain or an analog thereof, but is not limited thereto. Such information can also include information on diversity of interaction of a biomolecule or analog thereof such as a metabolite. "Genetic information" is also known as "nucleic acid information", which are synonymous.

As used herein, "biomolecule" refers to a molecule of any organism or virus. A biomolecule can include a nucleic acid, protein, sugar chain, lipid, and the like. As used herein, "analog of a biomolecule" refers to a naturally-occurring or non-naturally-occurring variant of a biomolecule. An analog of a biomolecule can include a modified nucleic acid, modified amino acid, modified lipid, modified sugar chain, and the like.

As used herein, "population" refers to a collection including two or more single biological units, cells, or cell-like constructs.

As used herein, "subpopulation", when used together with "population", refers to a portion of a population with fewer number of single biological units, cells, or cell-like constructs than the population.

As used herein, "gel" refers to a colloidal solution (sol) wherein a polymeric substance or colloidal particles form a mesh structure as a whole due to the interaction thereof, without fluidity while containing a large quantity of a liquid phase that is a solvent or dispersion medium. As used herein, "gelation" refers to changing a solution into a state of "gel".

As used herein, "capsule" refers to anything with a shape that can retain a cell or cell-like construct therein. As used herein, "gel capsule" refers to a gel-like microparticulate construct that can retain a cell or cell-like construct therein.

As used herein, "genetic analysis" refers to studying the state of a nucleic acid (DNA, RNA, or the like) in a biological sample. In one embodiment, genetic analysis includes those that utilize a nucleic acid amplification reaction. Examples of genetic analysis include, in addition thereto, sequencing, genotyping/polymorphism analysis (SNP analysis, copy number variation, restriction fragment length polymorphism, repeat number polymorphism), expression analysis, Quenching Probe (Q-Probe), SYBR green method, melt curve analysis, real-time PCR, quantitative RT-PCR, digital PCR, and the like.

As used herein, "single biological unit level" refers to processing genetic information or other information on a biomolecule contained in one single biological unit and genetic information or other information on a biomolecule contained in other single biological units in a distinguishable manner.

As used herein, "single cell level" refers to processing of genetic information or other information on a biomolecule contained in one cell or cell-like construct distinctly from genetic information or other information on a biomolecule contained in other cells or cell-like constructs. For example, when a polynucleotide is amplified at a "single biological unit level" or "single cell level", a polynucleotide in a single biological unit or a cell or cell-like construct and a polynucleotide in another single biological unit or cell or cell like unit are each amplified in distinguishable manner. In one embodiment of the present disclosure, a step of contacting said polynucleotide with an amplification reagent to amplify the polynucleotide within a gel capsule can also amplify while maintaining the polynucleotide in a gel state within a gel capsule.

As used herein, "single biological unit analysis" refers to analysis of genetic information or other information on a biomolecule contained in one single biological unit (e.g., cell or cell-like construct) distinctly from genetic information or other information on a biomolecule contained in other single biological units (e.g., cells or cell-like constructs).

As used herein, "single cell analysis" refers to analysis of genetic information or other information on a biomolecule contained in one cell or cell-like construct distinctly from genetic information or other information on a biomolecule contained in other cells or cell-like constructs.

As used herein, "genetic information" refers to information on a nucleic acid encoding a gene or other information contained in one cell or cell-like construct, including the presence/absence of a specific genetic sequence, yield of a specific gene, and total nucleic acid yield.

As used herein, "information on a biomolecule" refers to information on a biomolecule contained in one cell or cell-like construct (including nucleic acid as well as protein, sugar, lipid, and the like) or an analog thereof, including the presence/absence of a structure or sequence of a specific biomolecule, identity of a structure or sequence, yield of a specific biomolecule, and total biomolecule yield.

As used herein, "nucleic acid information" refers to information on a nucleic acid contained in one cell or cell-like construct, including the presence/absence of a specific genetic sequence, yield of a specific gene, and total nucleic acid yield.

As used herein, "identity" refers to similarity in structures or sequences between two biomolecules. If a sequence is targeted, identity can be determined by comparing positions in each sequence that can be aligned for comparison.

As used herein, "long-read sequencing" is a method of sequencing the entire sequence using a long read (a nucleotide chain that has been fragmented for analysis). In general, long-read sequencing performs decoding using a read with a length of 400 bases or longer.

### (Preferred Embodiments)

Preferred embodiments are described hereinafter. It is understood that the embodiments are exemplification of the present disclosure, and the scope of the present disclosure is not limited to such preferred embodiments. It is also understood that those skilled in the art can make appropriate modifications or changes within the scope of the invention by referring to the following preferred embodiments. Those skilled in the art can appropriately combine one or more of any of the embodiments.

### (Sequence information processing)

In one aspect, the present disclosure provides a method of processing sequence information on a single biological unit (e.g., cell or cell-like construct). The method comprises: (A) a step of clustering partial sequence information of sequence information of a plurality of single biological units (e.g., collection of genomes, transcriptomes, proteomes, equivalent genes, or the like) for each of the same lineages based on an organism lineage identification sequence (e.g., 16S rDNA or a marker gene); (B) optionally, a step of adding, to the cluster, partial sequence information on the single biological units corresponding to the cluster in a database; and (C) a step of creating a sequence information draft for the single biological units by using the partial sequence information of sequence information on the single biological units and sequence information on the single biological units in the database. Figure 1 shows an exemplary schematic diagram of this aspect. Figure **2** shows an example of the relationship between a draft genome and the single biological unit genome of the present disclosure.

Step (B) is an optional step, which may or may not utilize a database. In this manner, a clustering method can be a method utilizing a database (Figure **3**) or a method that does not utilize a database (Figure **4**)**.** If a database is utilized, partial sequence information on the single biological units corresponding to the cluster in a database is added to the cluster. If a database is not utilized, a cluster is newly created.

An organism lineage identification sequence (marker) can also be newly identified from a database after classification. In this aspect, the present disclosure provides a method of processing sequence information on a single biological unit (e.g., cell), the method comprising: (A) a step of clustering partial sequence information of sequence information on a plurality of single biological units for each of the same lineages based on an organism lineage identification sequence; and (B) a step of comparing partial sequence information corresponding to the cluster in a database with partial sequence information of the cluster, calculating a degree of similarity for each partial sequence, and identifying a partial sequence with a degree of similarity greater than or equal to a predetermined degree of similarity as an organism lineage identification sequence. In such a case, the organism lineage identification sequence can be used as a so-called biomarker. Figure **5** shows an exemplary schematic diagram of this aspect. In this aspect, a protein family of a registered draft genome is extracted to create a matrix of a draft genome and a corresponding protein family. The ratio of single copy genes with one corresponding protein family is calculated. A protein family that is present in the entire draft genome thereamong can be employed as a marker gene. The present disclosure provides a method of processing sequence information on a single biological unit, the method comprising: A) a step of extracting candidates of genes without duplication in a draft (single copy gene) from a database; B) a step of calculating the number (or a ratio) of corresponding drafts for each of the genes; and C) a step of sorting in ascending order of the number (or ratio) of the corresponding drafts and selecting a gene with a value greater than or equal to a predetermined value (or any number of genes from the highest ranking) as a marker gene candidate.

In one aspect, the present disclosure is a method of processing sequence information on a single biological unit, the method comprising: (D) a step of ranking partial sequence information of sequence information on a plurality of single biological units from a highest quality based on a predetermined judgment criterion (e.g., completion percentage or contamination percentage); (E) a step of selecting a population of a predetermined number of pieces of partial sequence information of sequence information on the plurality of single biological units from a highest ranking based on the ranking to construct a draft with a greater length (which can be a partial or full length) than the partial sequence information from the partial sequence information; and (E') a step of selecting a population of a set of a different number of pieces of partial sequence information of sequence information on the single biological units from the population, constructing a draft with a greater length than the partial sequence information from the partial sequence information, and selecting a draft created up to this point based on a predetermined judgment criterion (e.g., completion percentage or contamination percentage). It is preferable to repeat (E') because it is preferable to repeat draft creation while changing the number of SAGs. In some embodiments, the aforementioned (D) to (E') can be performed as a step for creating a sequence information draft of the single biological unit. Figure 7 shows a schematic diagram of this aspect.

In one preferred embodiment, the method of processing sequence information on a single biological unit of the present disclosure comprises: (F) a step of comparing the selected draft with partial sequence information of sequence information on a single biological unit that was not selected in (E) or (E') and selecting partial sequence information of sequence information on the single biological unit having a sequence of a portion that is not included in the draft; (G) a step of creating a longer draft by using the sequence information selected in (F) and the selected draft; (G') optionally, a step of repeating (G) preferably until the longer draft reaches a full length of sequence information; and (G") optionally, a step of repeating steps (D), (E), and (E') based on a judgment criterion with a lower criterion in the entire partial sequence information constituting the draft. For example, a looser parameter can be used as the judgment criterion with a lower criterion. Figure **8** shows a schematic diagram of this aspect.

In one aspect, the partial sequence information is SAG. In a specific aspect, the present disclosure provides a method of refining a cluster in an aspect related to the stage immediate after determining that SAG is of the "same" cluster (e.g., lineage or species). In this aspect, the present disclosure is a method of processing sequence information on a single biological unit, the method comprising: (A) a step of clustering partial sequence information of sequence information on a plurality of single biological units for each of the same lineages based on an organism lineage identification sequence; (H) a step of evaluating partial sequence information of sequence information on the plurality of single biological units constituting sequence information on a single biological unit based on an organism lineage identification sequence within the cluster of the same lineage, and reclustering within the cluster of the same lineage; (H') a step of comparing a sequence information draft created from the cluster of the same lineage with a sequence information draft created from the reclustered cluster; and (I) a step of determining whether reclustering in (H) is appropriate for a result of comparison based on a predetermined judgment criterion (e.g., completion percentage or contamination percentage), and, if appropriate, registering the draft in a database as a new group.

In this regard, the evaluation described above can evaluate extracted partial sequence information (e.g., SAGs) with a marker gene in a round robin format, and the evaluation can use, for example, the distance between each SAG.

In a preferred embodiment, reclustering in the present disclosure is performed through network analysis and community detection.

The present disclosure also provides processing in an aspect of the stage after draft quality no longer improves even after increasing the number of pieces of partial sequence information (e.g., SAGs). In this aspect, the present disclosure is a method of processing sequence information on a single biological unit, the method comprising: (A) a step of clustering partial sequence information of sequence information on a plurality of single biological units for each of the same lineages based on an organism lineage identification sequence; (D) a step of ranking partial sequence information of sequence information on the plurality of single biological units belonging to the cluster of the same lineage from a highest quality based on a predetermined judgement criterion (e.g., completion percentage or contamination percentage); (E) a step of selecting a population of a predetermined number of pieces of partial sequence information of sequence information on the plurality of single biological units from a highest ranking based on the ranking to construct a draft with a greater length (which can be a partial or full length) than the partial sequence information from the partial sequence information; (E") a step of selecting a population of a set of a different number of pieces of partial sequence information of sequence information on the single biological unit from the population, constructing a draft with a greater length than the partial sequence information from the partial sequence information, and evaluating a draft created up to this point based on a predetermined judgment criterion (e.g., completion percentage or contamination percentage); (H) a step of evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change (i.e., remains within a certain range) due to an increase in the number in a population of a set of sequence information; (H') a step of comparing a sequence information draft created from the cluster of the same lineage with a sequence information draft created from the reclustered cluster; and (J) a step of determining whether reclustering in (H) is appropriate for a result of comparison based on a predetermined judgment criterion (e.g., completion percentage or contamination percentage), and, if appropriate, repeating (D) to (E') for partial sequence information of sequence information on the plurality of single biological units belonging to the reclustered cluster.

It is understood that each of the steps in these method can be appropriately combined in the present disclosure. When processing sequence information on a single biological unit and screening for candidates of an organism lineage identification sequence in some embodiments, the location from which an instruction to execute them is given to a computer can be different from the location where the instruction is received to actually perform these processing or the like. In another embodiment, each processing of the method of the present disclosure can be executed by a computer. In another embodiment, the database of the present disclosure can be a database created by the clustering or sequence analysis method of the present disclosure or a database created independently from the clustering or sequence analysis method of the present disclosure. In a preferred embodiment, a database created independently from the clustering or sequence analysis method of the present disclosure can be a database for data obtained by sequencing a sequence that is amplified based on single cell amplification. While it was understood that addition of a sequence in another database would lead to reduced quality in conventional art, it was found that the quality of sequences actually improves by adding a sequence in another database to a cluster.

In some embodiments where a draft genome is constructed from sequence data, a certain amount of partial sequence information comprising a sequence site found to have a large number of duplicate readings can be removed to correct (homogenize) a bias in sequence reads. Further improvement in genome quality is expected by repeated homogenization using a genome sequence created from homogenized sequence data as a reference sequence in response to clustering of sequence data that has been homogenized. If partial sequence information subjected to homogenization processing is read by long-read sequencing, even further improvement in genome quality is expected.

If a draft genome of a sequence derived from a single biological unit is constructed, this presumes that data itself is clean and has a certain degree of genome integrity, and a plurality of pieces of single cell data are obtained together. This could not be materialized with conventional art, but was materialized for the first time by the present disclosure. Further, a draft genome of a sequence derived from a single biological unit was never decoded by long-read sequencing. Since it was understood that a sequence derived from a single biological unit has a problem of producing a chimera (separate genome sequences that are not inherently connected are generated due to an error during amplification or the like to produce incorrectly decoded sequence data), a long-read assembly system that is suitable for single cell data with a chimera and high amplification bias was not developed. Such a bias can be drastically reduced by referring to a plurality of single cell genomes and repeating mapping and assembly by utilizing the present disclosure. This allows an extremely accurate genome sequence to be obtained.

It is well known that a bias is generated in a sequence of an amplified DNA such as a genome sequence derived from a single cell. In this regard, homogenization processing (for reducing bias) in conventional methods designs enzymatic reactions or reaction conditions so that bias itself is not likely to occur upon amplification (Nishikawa et al. PLoS ONE), or uses a method of proactively degrading a DNA to reduce a bias generated after amplification or the like. However, a problem with these methods was that biases could not be completely removed. Since the present disclosure executes in silico processing even on data with a bias, data can be homogenized without the special designs in the reaction system described above. Since it is presumed that data itself is clean and is derived from a plurality of origins, this could only be executed by the method utilized in the present disclosure. For accuracy of a genome sequence, conventional methods perform mapping on a reference genome of related species or the like and evaluate a bias, gap, etc., to correct the sequence. Meanwhile, the method utilized in the present disclosure achieves a particularly significant effect compared to conventional art in that data for an unknown microorganism sample without a reference sequence can also be homogenized because self data can be referenced to execute homogenization processing by comprehensively analyzing a plurality of pieces of data for the same species even without a relative species reference genome upon homogenization processing. Further, the method is extremely effective in decoding the complete genome of an unknown microorganism. The method can also decode a gene cluster by the entire sequence without a gap, without culturing, in cells wherein a gene cluster position in the genome has not been identified, and the function thereof can be understood in detail. Further, research and development that introduce the gene cluster into another organism that can be readily handled to create an intended substance is also possible. The following application examples/envisioned examples are expected.
*surveillance of antibiotic resistant gene/resistant strain
*finishing of a microorganism genome sequence (closing as a circular genome) (basically, closing was rarely possible in strains other than cultured strains)
*acquisition of biosynthesized gene cluster
*evaluation of gene alteration of a microorganism host by synthetic biology
*genomic structure mutation and evaluation of effect on various metabolic function/host organism

### (Program and recording medium)

In one aspect, the present disclosure provides a computer program for instructing a computer to implement a method of processing sequence information on a single biological unit (e.g., cell or cell-like construct) and a recording medium for storing the program (e.g., CD-R, flash memory, hard disk, transmission medium, cloud, or the like). The method implemented by the program comprises: (A) a step of clustering partial sequence information of sequence information on a plurality of single biological units (e.g., collection of genomes, transcriptomes, proteomes, equivalent genes, or the like) for each of the same lineages based on an organism lineage identification sequence (e.g., 16S rDNA or a marker gene); (B) optionally, a step of adding, to the cluster, partial sequence information on the single biological units corresponding to the cluster in a database; and (C) a step of creating a sequence information draft for the single biological units by using the partial sequence information of sequence information on a single biological units and sequence information on the single biological units in the database. Figure **1** shows an exemplary schematic diagram of this aspect. Figure **2** shows an example of the relationship between a draft genome and the single biological unit genome of the present disclosure.

Step (B) is an optional step, which may or may not utilize a database. In this manner, a clustering method can be a method utilizing a database (Figure **3**) or a method that does not utilize a database (Figure **4**). If a database is utilized, partial sequence information on the single biological units corresponding to the cluster in a database is added to the cluster. If a database is not utilized, a cluster is newly created.

An organism lineage identification sequence (marker) can also be newly identified from a database after classification. In this aspect, the present disclosure provides a computer program for instructing a computer to implement a method of processing sequence information on a single biological unit (e.g., cell) and a recording medium for storing the program (e.g., CD-R, flash memory, hard disk, transmission medium, cloud, or the like). The method implemented by the program comprises: (A) a step of clustering partial sequence information of sequence information on a plurality of single biological units for each of the same lineages based on an organism lineage identification sequence; and (B) a step of comparing partial sequence information corresponding to the cluster in a database with partial sequence information of the cluster, calculating a degree of similarity for each partial sequence, and identifying a partial sequence with a degree of similarity greater than or equal to a predetermined degree of similarity as an organism lineage identification sequence. In such a case, an organism lineage identification sequence can be used as a so-called biomarker. Figure **5** shows an exemplary schematic diagram of this aspect. In this aspect, a protein family for a registered draft genome is extracted to create a matrix of a draft genome and a corresponding protein family. The ratio of single copy genes with one corresponding protein family is calculated. A protein family that is present in the entire draft genome thereamong can be employed as a marker gene. The present disclosure provides a program for instructing a computer to implement a method of processing sequence information on a single biological unit and a recording medium for storing the program, the method comprising: A) a step of extracting candidates of genes without duplication in a draft (single copy gene) from a database; B) a step of calculating the number (or a ratio) of corresponding drafts for each of the genes; and C) a step of sorting in ascending order of the number (or ratio) of the corresponding drafts and selecting a gene with a value greater than or equal to a predetermined value (or any number of genes from the highest ranking) as a marker gene candidate.

In one aspect, the present disclosure provides a computer program for instructing a computer to implement a method of processing sequence information on a single biological unit and a recording medium for storing the program (e.g., CD-R, flash memory, hard disk, transmission medium, cloud, or the like). The method implemented by the program comprises: (D) a step of ranking partial sequence information of sequence information on a plurality of single biological units from a highest quality based on a predetermined judgment criterion (e.g., completion percentage or contamination percentage); (E) a step of selecting a population of a predetermined number of pieces of partial sequence information of sequence information on the plurality of single biological units from a highest ranking based on the ranking to construct a draft with a greater length (which can be a partial or full length) than the partial sequence information from the partial sequence information; and (E') a step of selecting a population of a set of a different number of pieces of partial sequence information of sequence information on the single biological units from the population, constructing a draft with a greater length than the partial sequence information from the partial sequence information, and selecting a draft created up to this point based on a predetermined judgment criterion (e.g., completion percentage or contamination percentage). It is preferable to repeat (E') because it is preferable to repeat draft creation while changing the number of SAGs. Figure **7** shows a schematic diagram of this aspect.

In a preferred embodiment, a method of processing sequence information on a single biological unit implemented by the program of the present disclosure comprises: (F) a step of comparing the selected draft with partial sequence information of sequence information on a single biological unit that was not selected in (E) or (E') and selecting partial sequence information of sequence information on the single biological unit having a sequence of a portion that is not included in the draft; (G) a step of creating a longer draft by using the sequence information selected in (F) and the selected draft; (G') optionally, a step of repeating (G) preferably until the longer draft reaches a full length of sequence information; and (G") optionally, a step of repeating steps (D), (E), and (E') based on a judgment criterion with a lower criterion in the entire partial sequence information constituting the draft. For example, a looser parameter can be used as the judgment criterion with a lower criterion. Figure **8** shows a schematic diagram of this aspect.

In another aspect, the program of the present disclosure encodes a method of refining a cluster in an aspect related to the stage immediately after determining that SAG is of the "same" cluster (e.g., lineage or species). In this aspect, the present disclosure provides a computer program for instructing a computer to implement a method of processing sequence information on a single biological unit and a recording medium for storing the program (e.g., CD-R, flash memory, hard disk, transmission medium, cloud, or the like). The method implemented by the program comprises: (A) a step of clustering partial sequence information of sequence information on a plurality of single biological units for each of the same lineages based on an organism lineage identification sequence; (H) a step of evaluating partial sequence information of sequence information on the plurality of single biological units constituting sequence information on a single biological unit based on an organism lineage identification sequence within the cluster of the same lineage, and reclustering within the cluster of the same lineage; (H') a step of comparing a sequence information draft created from the cluster of the same lineage with a sequence information draft created from the reclustered cluster; and (I) a step of determining whether reclustering in (H) is appropriate for a result of comparison based on a predetermined judgment criterion (e.g., completion percentage or contamination percentage), and, if appropriate, registering the draft in a database as a new group.

In this regard, the evaluation described above can evaluate extracted partial sequence information (e.g., SAGs) with a marker gene in a round robin format, and the evaluation can use, for example, the distance between each SAG. In a preferred embodiment, reclustering in the present disclosure is performed through network analysis and community detection.

The program of the present disclosure also provides processing in an aspect of the stage after draft quality no longer improves even after increasing the number of pieces of partial sequence information (e.g., SAGs). In this aspect, the present disclosure provides a computer program for instructing a computer to implement a method of processing sequence information on a single biological unit and a recording medium for storing the program (e.g., CD-R, flash memory, hard disk, transmission medium, cloud, or the like). The method implemented by the program comprises: (A) a step of clustering partial sequence information of sequence information on a plurality of single biological units for each of the same lineages based on an organism lineage identification sequence; (D) a step of ranking partial sequence information of sequence information on the plurality of single biological units belonging to the cluster of the same lineage from a highest quality based on a predetermined judgment criterion (e.g., completion percentage or contamination percentage); (E) a step of selecting a population of a predetermined number of pieces of partial sequence information of sequence information on the plurality of single biological units from a highest ranking based on the ranking to construct a draft with a greater length (which can be a partial or full length) than the partial sequence information from the partial sequence information; (E") a step of selecting a population of a set of a different number of pieces of partial sequence information of sequence information on the single biological unit from the population, constructing a draft with a greater length than the partial sequence information from the partial sequence information, and evaluating a draft created up to this point based on a predetermined judgment criterion (e.g., completion percentage or contamination percentage); (H) a step of evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change (i.e., remains within a certain range) due to an increase in the number in a population of a set of sequence information; (H') a step of comparing a sequence information draft created from the cluster of the same lineage with a sequence information draft created from the reclustered cluster; and (J) a step of determining whether reclustering in (H) is appropriate for a result of comparison based on a predetermined judgment criterion (e.g., completion percentage or contamination percentage), and, if appropriate, repeating (D) to (E') for partial sequence information of sequence information on the plurality of single biological units belonging to the reclustered cluster.

In another aspect, the present disclosure provides a data structure containing partial sequence information of sequence information on a plurality of single biological units clustered for each of the same lineages based on an organism lineage identification sequence. In one embodiment, the partial sequence information contained in the data structure is derived from two or more independently clustered and created databases. In one embodiment, information associated with the independently performed clustering is linked to and stored with the partial sequence information. In one embodiment, the partial sequence information, as a whole, constitutes genomic information. In one embodiment, the partial sequence information is collected for each single biological unit. In one embodiment, the partial sequence information is linked to and stored with identification information (ID information) on a single biological unit from which the partial sequence information is derived.

In another embodiment, the present disclosure provides a data structure for a single biological unit from integrating a plurality of data structures, containing partial sequence information of sequence information on a plurality of single biological units clustered for each of the same lineages based on an organism lineage identification sequence. A high quality database integrating a single biological unit such as a single cell was not available in the past, and is provided for the first time by the present disclosure.

### (System)

In one aspect, the present disclosure provides a system for processing sequence information on a single biological unit (e.g., cell or cell structure). The system comprises:
(A) a clustering unit for clustering partial sequence information of sequence information on a plurality of single biological units (e.g., collection of genomes, transcriptomes, proteomes, equivalent genes, or the like) for each of the same lineages based on an organism lineage identification sequence (e.g., 16S rDNA or a marker gene);
(B) optionally, an additional information addition unit for adding, to the cluster, partial sequence information on the single biological units corresponding to the cluster in a database (this can be the same or separate from the clustering unit); and (C) a draft creation unit for creating a sequence information draft for the single biological units by using the partial sequence information of sequence information on the single biological units and sequence information on the single biological units in the database. Figure 1 shows an exemplary schematic diagram of this aspect. Figure **2** shows an example of the relationship between a draft genome and the single biological unit genome of the present disclosure.

The additional information addition unit corresponding to B) is optional, which may or may not utilize a database.

In this manner, a clustering method materialized by the clustering unit can be a method utilizing a database (Figure **3****)** or a method that does not utilize a database (Figure **4****).** If a database is utilized, partial sequence information on the single biological unit corresponding to the cluster in a database is added to the cluster. If a database is not utilized, a cluster is newly created.

The system of the present disclosure can newly identify an organism lineage identification sequence (marker) from a database after classification. In this aspect, the present disclosure provides a system for processing sequence information on a single biological unit (e.g., cell). The system comprises: (A) a clustering unit for clustering partial sequence information of sequence information on a plurality of single biological units for each of the same lineages based on an organism lineage identification sequence; and (B) an identification unit (also referred to as marker identification unit) for comparing partial sequence information corresponding to the cluster in a database with partial sequence information of the cluster, calculating a degree of similarity for each partial sequence, and identifying a partial sequence with a degree of similarity greater than or equal to a predetermined degree of similarity as an organism lineage identification sequence. In such a case, an organism lineage identification sequence can be used as a so-called biomarker. Figure **5** shows an exemplary schematic diagram of this aspect. In this aspect, a protein family for a registered draft genome is extracted to create matrix of a draft genome and a corresponding protein family. The ratio of single copy genes with one corresponding protein family is calculated. A protein family that is present in the entire draft genome thereamong can be employed as a marker gene. The present disclosure provides a system for processing sequence information on a single biological unit, the system comprising: A) an extraction unit for extracting genes without duplication from a draft in a database; B) a calculation unit for calculating the number or a ratio of corresponding drafts for each of the genes; and C) a selection unit for selecting a gene with the number or ratio of corresponding drafts greater than or equal to a predetermined value as a candidate of an organism lineage identification sequence.

In one aspect, the present disclosure provides a system for processing sequence information on a single biological unit. The system comprises: (D) a ranking unit for ranking partial sequence information of sequence information on a plurality of single biological units from a highest quality based on a predetermined judgment criterion (e.g., completion percentage or contamination percentage); and (E) a draft construction unit for selecting a population of a predetermined number of pieces of partial sequence information of sequence information on the plurality of single biological units from a highest ranking based on the ranking to construct a draft with a greater length (which can be a partial or full length) than the partial sequence information from the partial sequence information, selecting a population of a set of a different number of pieces of partial sequence information of sequence information on a single biological unit from the population, constructing a draft with a greater length than the partial sequence information from the partial sequence information, and selecting a draft created up to this point based on a predetermined judgment criterion (e.g., completion percentage or contamination percentage). It is preferable to repeat draft creation a plurality of times because it is preferable to repeat draft creation while changing the number of pieces of partial sequence information (e.g., SAGs). Figure **7** shows a schematic diagram of this aspect.

In a preferred embodiment, the system of the present disclosure comprises: (F) a selection unit for comparing the selected draft with partial sequence information of sequence information on a single biological unit that was not selected in (E) or (E') and selecting partial sequence information of sequence information on the single biological unit having a sequence of a portion that is not included in the draft (this can be configured as a part of the draft construction unit); (G) a draft improvement unit for creating a longer draft by using the sequence information selected in (F) and the selected draft (this can also be configured as a part of the draft construction unit); (G') optionally, a draft construction unit for repeating (G) preferably until the longer draft reaches a full length of sequence information; and (G") optionally, means for repeating the ranking, draft construction, and selection in (D), (E), and (E') based on a judgment criterion with a lower criterion in the entire partial sequence information constituting the draft. The repeat can be materialized in the draft construction unit or the like. For example, a looser parameter can be used as the judgment criterion with a lower criterion. Figure **8** shows a schematic diagram of this aspect.

In another aspect, the system of the present disclosure encodes a method of refining a cluster in an aspect related to the stage immediately after determining that SAG is of the "same" cluster (e.g., lineage or species). In this aspect, the present disclosure provides a system for processing sequence information on a single biological unit. The system comprises: (A) a clustering unit for clustering partial sequence information of sequence information on a plurality of single biological units for each of the same lineages based on an organism lineage identification sequence; (H) a reclustering unit for evaluating partial sequence information of sequence information on the plurality of single biological units constituting sequence information on a single biological unit based on an organism lineage identification sequence within the cluster of the same lineage, and reclustering within the cluster of the same lineage (this can be materialized in the clustering unit); (H') a comparison unit for comparing a sequence information draft created from the cluster of the same lineage with a sequence information draft created from the reclustered cluster (this can also be materialized in the clustering unit); and (I) a registration unit for determining whether reclustering in (H) is appropriate for a result of comparison based on a predetermined judgment criterion (e.g., completion percentage or contamination percentage), and, if appropriate, registering the draft in a database as a new group.

In this regard, the evaluation described above can evaluate extracted partial sequence information (e.g., SAGs) with a marker gene in a round robin format, and the evaluation can use, for example, the distance between each SAG.

In a preferred embodiment, reclustering in the present disclosure is performed through network analysis and community detection.

The system of the present disclosure also provides processing in an aspect of the stage after draft quality no longer improves even after increasing the number of partial sequence information (e.g., SAGs). In this aspect, the present disclosure provides a system for processing sequence information on a single biological unit. The system comprises: (A) a clustering unit for clustering partial sequence information of sequence information on a plurality of single biological units for each of the same lineages based on an organism lineage identification sequence; (D) a ranking unit for ranking partial sequence information of sequence information on the plurality of single biological units belonging to the cluster of the same lineage from a highest quality based on a predetermined judgment criterion (e.g., completion percentage or contamination percentage); (E) a draft construction unit for selecting a population of a predetermined number of pieces of partial sequence information of sequence information on the plurality of single biological units from a highest ranking based on the ranking to construct a draft with a greater length (which can be a partial or full length) than the partial sequence information from the partial sequence information, selecting a population of a set of a different number of pieces of partial sequence information of sequence information on the single biological unit from the population, constructing a draft with a greater length than the partial sequence information from the partial sequence information, and evaluating a draft created up to this point based on a predetermined judgment criterion (e.g., completion percentage or contamination percentage); (H) a reclustering unit for evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change (i.e., remains within a certain range) due to an increase in the number in a population of a set of sequence information (this can be materialized in the clustering unit); (H') a comparison unit for comparing a sequence information draft created from the cluster of the same lineage with a sequence information draft created from the reclustered cluster (this can also be materialized in the clustering unit); and (J) a determination unit for determining whether reclustering in (H) is appropriate for a result of comparison based on a predetermined judgment criterion (e.g., completion percentage or contamination percentage), and, if appropriate, the determination unit repeats the steps of (D) to (E') forpartial sequence information of sequence information on the plurality of single biological units belonging to the reclustered cluster.

The system, program, recording medium, and method according to one or more embodiments of the present disclosure have been described based on the embodiments, but the present disclosure is not limited to such embodiments. Various modifications applied to the present embodiments and embodiments constructed by combining constituent elements in different embodiments that are conceived by those skilled in the art are also encompassed within the scope of one or more embodiments of the present disclosure, as long as such embodiments do not deviate from the intent of the present disclosure.

Some or all of the constituent elements of the present disclosure in each of the embodiments described above can be comprised of a single system LSI (Large Scale Integration). For example, the system for processing sequence information of the present disclosure can be optionally combined with a database, or can be equipped with or combined with a system for identifying a sequence with a function such as a biomarker (Figure **10****).**

System LSI is ultra-multifunctional LSI manufactured by integrating a plurality of constituents on a single chip, or specifically, a computer system comprised of a microprocessor, ROM (Read Only Memory), RAM (Random Access Memory), and the like. A computer program is stored in a ROM. The system LSI accomplishes its function by the microprocessor operating in accordance with the computer program. The term system LSI is used herein, but the term IC, LSI, super LSI, and ultra LSI can also be used depending on the difference in the degree of integration. The method for forming an integrated circuit is not limited to LSI. An integrated circuit can be materialized with a dedicated circuit or universal processor. After the manufacture of LSI, a programmable FPGA (Field Programmable Gate Array) or reconfigurable processor which allows reconfiguration of the connection or setting of circuit cells inside the LSI can be utilized. If a technology of integrated circuits that replaces LSI by advances in semiconductor technologies or other derivative technologies becomes available, functional blocks can obviously be integrated using such technologies. Application of biotechnology or the like is a possibility.

One aspect of the present disclosure can be not only such a sequence information processing device or system, but also a functionally specialized system (e.g., biomarker screening device, efficacy determination device, diagnostic device, etc.). Further, one embodiment of the present disclosure can be a computer program causing a computer to execute each characteristic step in sequence information processing. One embodiment of the present disclosure can also be a computer readable non-transient recording medium on which such a computer program is recorded.

In each of the embodiments described above, each constituent element can be materialized by being composed of a dedicated hardware or by executing a software program that is suited to each constituent element. Each constituent element can be materialized by a program execution unit such as a CPU or a processor reading out and executing a software program recorded on a recording medium such as a hard disk or semiconductor memory. In this regard, a software materializing the present disclosure of each of the embodiments described above or the like can be a program such as those described above herein.

### (Embodiment using cloud, IoT, and AI)

The sequence information processing technology of the present disclosure can be provided in a form comprising all constituents as a single system or device. Alternatively, the technology can also be envisioned in a form of mainly displaying analysis and results as a sequence information processing device while calculation or differentiation model calculation is performed on a server or cloud. Some or all of them can be performed using IoT (Internet of Things) and/or artificial intelligence (AI) (Figure **11****).**

Alternatively, a sequence information processing device can also be envisioned in a semi-standalone form where means required for various calculations is stored and performs an analysis therein, but the calculations required for the analysis are performed on a server or cloud. Since transmission/reception is not always possible at some locations such as hospitals, this is a model envisioned for use when communication is blocked.

A storage unit can be a recording medium such as a CD-R, DVD, Blu-ray, USB, SSD, or hard disk. A storage unit can be stored in a server or in a form of appropriately recording on the cloud.

"Software as a service (SaaS)" mostly falls under such a cloud service. Since a sequence information processing device is understood to be installed with a differentiation algorithm made from data produced in a laboratory environment, the device can be provided as a system comprising two or three features of these embodiments.

Data can also be stored as needed. Data storage is generally equipped on the server side, but data storage can be at the terminal side for not only fully equipped models but also for cloud models (optional). When a service is provided on the cloud, options such as standard (e.g., up to 10 Gb on the cloud), option 1 (e.g., additional 10 Tb on the cloud), option 2 (parameter is set for divided storage on the cloud), and option 3 (analysis optionally stored on the cloud) can be provided for data storage. Data is stored, and data is imported from all sold devicees to create big data (e.g., sequence database), and an analysis model is continuously updated or a new model is constructed so that new differentiation model software such as "disease determination model" can be provided.

There can also be data analysis options. In this regard, request of a user of a service provider or the like can be provided. In other words, this can be envisioned as an option for a calculation method.

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. While the present disclosure is described hereinafter based on Examples, the above descriptions and the following Examples are not provided to limit the present disclosure, but for the sole purpose of exemplification. Thus, the scope of the present disclosure is not limited to the embodiments or the Examples specifically described herein and is limited only by the scope of claims.

### [Examples]

The Examples are described hereinafter.

For reagents, the specific products described in the Examples were used. However, an equivalent product from another manufacturer can also be used instead.

### (Example 1)

### (Draft genome creation method)

### (Method)

### (Cell line sample preparation)

12 SAG data each for E. coli K12 (ATCC 10798) and B. subtilis (ATCC 6633) were obtained from Hosokawa et al. In the paper of Hosokawa et al., these cells were acquired from the ATCC. E. coli K12 was cultured in Luria-Bertani (LB) medium (1.0% Bacto-tryptone, 0.5% yeast extract, 1.0% NaCl, pH 7.0). B. subtilis was cultured in Brain Heart Infusion Broth (ATCC medium 44, Thermo Fisher Scientific, San Jose, CA, USA). The collected cells were washed three times with UV-treated Phosphate-Buffered Saline (-) (PBS, Thermo Fisher Scientific) and subjected to single-droplet MDA and sequencing.

### (Preparation of mouse gut microbiota)

Feces was collected from a male 7-week-old ICR mouse (Tokyo Laboratory Animals Science Co., Ltd., Tokyo, Japan) and homogenized in PBS. The supernatant was recovered by centrifugation at 2000 × g for 2 seconds, and centrifuged at 15000 × g for 3 minutes. The resulting cell pellet was washed twice with PBS, and finally resuspended in PBS.

### (Single droplet MDA)

A microfluidic droplet generator and an MDA reaction device were fabricated and used for single-droplet MDA according to the report of Hosokawa et al. Prior to analysis, cell suspensions were adjusted to a concentration of 0.1 cells/droplet to prevent encapsulation of multiple cells in a single droplet. Using the droplet generator, single microbial cells were encapsulated in lysis buffer D2 (QIAGEN, Hilden, Germany), and lysed at 65°C for 10 minutes. Cell lysates were then injected into a droplet fusion device and mixed with droplets of MDA reaction mix (REPLI-g Single Cell Kit, QIAGEN) supplemented with Tween-20 and EvaGreen. After collection in PCR tubes, the droplets were incubated at 30°C for 2 hours and at 65°C for 3 minutes. For single-cell sequencing, droplets that became fluorescent were individually picked and transferred by micropipette under an open clean bench (KOACH 500-F, KOKEN LTD., Tokyo, Japan) into fresh MDA reaction mix. After 2 hours of incubation at 30°C, the enzyme was inactivated at 65°C for 3 minutes.

### (16S rDNA sequencing)

To confirm amplification from single cells, 16S rRNA gene fragments V3-V4 were amplified and sequenced by Sanger sequencing from SAGs obtained by single-droplet MDA. To compare the phylogenetic distribution, 16S rRNA fragments (V3-V4) were also amplified from a metagenomic sample of gut microbiota and sequenced by MiSeq (Illumina, San Diego, CA, USA). Paired-end reads were connected, trimmed, and clustered by UPARSE into taxonomic units at 97% identity. Taxonomy was determined in RDP classifier.

### (Library preparation and whole genome sequencing)

Illumina libraries for single-cell sequencing were prepared from products of single-droplet MDA using Nextera XT DNA sample prep kit (Illumina) with Nextera XT Index Kit. Libraries were then sequenced on an Illumina MiSeq system at 2 × 300 paired-end reads.

### (Quality control of SAG reads and construction of cross-reference contigs (step 1 in ccSAG))

SAGs were first grouped based on 16S rRNA similarity ≧ 99% and ANI ≧ 95%. Nucleotide identity was estimated by pairwise BLAST between full-length raw SAG contigs, and was calculated over ≧ 500 bp. Grouped SAG reads were then prefiltered using FASTX-toolkit (http://hannonlab.cshl.edu/fastx_toolkit/) and PRINSEQ to remove low-quality reads (≧ 50% of bases with quality scores < 25), trim the 3'-end of reads with low-quality bases (quality score < 20), remove short reads (<20 bp) and reads with 1% of bases unidentified, and discard unpaired reads after such prefiltration. Subsequently, contigs were individually assembled de novo from raw SAG reads using SPAdes-3.9.0 with options-careful-disable-rr-sc. Finally, raw SAG contigs ≧ 500 bp were collected for cross-reference mapping.

### (Removal of chimeric reads by cross reference mapping (step 2 of ccSAG))

Quality-controlled reads from one SAG were mapped by BWA to multiple raw contigs constructed from other SAGs in the same group. A read was considered clean if complete alignment to reference contigs was equally or more frequent than partial alignment (soft clipping), but considered potentially chimeric if partial alignment was more frequent than complete alignment. Potential chimeras were then split into aligned and unaligned fragments, which were then remapped to multiple raw contigs and reclassified as described. Finally, fully unaligned reads and fragmented chimeras shorter than 20 bp were discarded as unmapped. Cycles of cross-reference mapping and chimera splitting were repeated until partially aligned, potentially chimeric reads were undetectable.

### (Co-assembly of clean SAGs and contig extension (step 3 in ccSAG))

Clean reads from each SAG were co-assembled de novo using SPAdes into clean composite SAG contigs. Similarly, raw SAG reads were co-assembled de novo into raw composite SAG contigs. Gaps between clean composite contigs were filled by BLAST mapping against raw composite contigs. Briefly, potentially usable raw composite contigs were identified by ≧ 99% identity to clean composite contigs. Such raw composite contigs were then collected into a database, against which clean composite contigs were mapped by BLAST and gap-filled based on the resulting alignments, thereby generating bridged composite SAG contigs, which essentially comprise the composite single-cell genome.

### Analysis of SAG assembly:

Assembly quality was evaluated by QUAST (Gurevich A et al., Bioinformatics. 2013 Apr 15; 29(8):1072-5.). For the analysis of cell lines, all sequence data were mapped to the NCBI reference genome of NC_00913 (E. coli substrain MG1655) with f-plasmid and lambda phage sequence or NCBI reference genome of NC_014479 (Bacillus subtilis subsp. Spizizenii str. W23). For the analysis of uncultured cell genomes obtained by this Example, bridged composite SAG contigs were used as references to identify potential misassemblies and determine the genome fraction of each SAG. Completeness and contamination were evaluated by CheckM (Parks DH et al., Genome Res. 2015 Jul; 25(7):1043-55.). Taxonomy was assigned in AMPHORA2 or by BLAST search of 16S rDNA sequences in RNAmmer (Lagesen K et al., Nucleic Acids Res. 2007; 35(9):3100-8.). Gene pathway analysis was performed in KAAS (Moriya Y et al., Nucleic Acids Res. 2007 Jul; 35 (Web Server issue):W182-5.) and MAPLE (Takami H et al., DNA Res. 2016 Jul 3. pii: dsw030.), while assembly graphs were generated in Bandage (Wick RR et al., Bioinformatics. 2015 Oct 15; 31(20): 3350-2.). For the analysis of SNPs, each single-cell-amplified genome was mapped onto the coding sequences of the bridged composite SAG contigs, and then the nucleotides were screened for sites with a coverage depth of at least 5 reads where 99.9% of reads did not match the reference and showed homogeneous bases (nucleic acid sequence). After that, nucleotide sites that contained both multiple matched SAGs and unmatched SAGs in same strains were identified as SNPs.

### (Example 2) Microorganism genome database construction

Figure **2** is the configuration of a microorganism genome database for use in the present disclosure. An Example of constructing a microorganism genome database with a relational database system is shown therein. An operating system and a relational database management system are installed on a computer system consisting primarily of a central processing unit (CPU), primary storage device, auxiliary storage device, input/output device, and other peripheral devices. As shown in Figure **2****,** tables for storing draft genomic information and corresponding assembled base sequence, genetic information, and gene base sequence, and single biological unit genomic information and corresponding assembled base sequence, genetic information, gene base sequence, and partial base sequence information are created on the relational database. Assembled base sequence, gene base sequence, and partial base sequence can also be in a form where actual data is stored outside of the relational database and references to the actual data are stored in the tables. A column for storing information associated with the draft genome is created in the draft genomic information table. Examples include provisional phylogenetic classification, completion percentage, contamination percentage, quality category, genome size, contig count, N50 value, GC content, and the like. Provisional phylogenetic classification is information on the phylogenetic classification of an organism obtained by analyzing an assembled base sequence with a dedicated analysis tool (e.g., checkM, or the like). This can identify the rough lineage of the draft genome. The quality category is for indicating the state of the draft genome. It is desirable that this is a category in accordance with an international specification or the like. A column for storing information associated with a gene of a draft genome is created in the gene information table for the draft genome. Examples include name of gene, gene length, protein family, GC content, marker type, single copy, and the like. Protein family is information obtained by homology analysis with a protein database or the like. Marker type is for denoting whether it is a marker for use in phylogenetic classification or the like. Single copy represents whether a gene is a single copy gene. A single copy gene can be identified by the method in Figure **5****.** A column for storing information associated with a single biological unit genome is created in the single biological unit genomic information table. Examples include provisional phylogenetic classification, completion percentage, contamination percentage, genome size, contig count, N50 value, GC content, and the like. A column for storing information associated with a gene of a single biological unit genome is created in the genetic information table for the single biological unit genome. Examples include name of gene, gene length, protein family, GC content, single copy, and the like. A column for storing the base sequence (reference thereof if stored as an external file) is created in each of the other base sequence tables. It is preferable to prepare an ID column for uniquely identifying data in each table. Draft genomic information and single biological unit genomic information have a one to many relationship. Genomic information and assembled base sequence have a one to many relationship. Genomic information and genetic information have a one to many relationship. Genetic information and gene base sequence have a one to one relationship. Single biological unit genomic information and partial base sequence have a one to many relationship.

### (Example 3) Clustering single biological unit genome using a microorganism genome database

Figure **3** shows a method of organizing partial base sequences of a single biological unit that have been obtained as a cluster of the same lineage. It is assumed, for example, that genome DNA of a single biological unit was analyzed with Illumina's DNA sequencer or the like, and a base sequence file in a fastq format or the like was obtained. A partial base sequence is described in the fastq file. The obtained fastq file is stored in a computer for analysis. The partial base sequences within the fastq file include partial base sequences with an adaptor sequence and low quality partial base sequences. Such low quality partial base sequences are deleted using a quality control tool such as fastqc. The fastq file is assembled with an assembly tool such as Spades to obtain an assembled base sequence. For the assembled base sequence, evaluation values such as the contig count, genome size, completion percentage, or contamination percentage are calculated using an evaluation tool such as QUAST or checkM. A gene is then identified from the assembled base sequence. A gene annotation tool such as Prokka or DFAST is used for identifying a gene. For the identified gene base sequence, functional information such as protein family can be obtained by searching a protein database such as Pfam. Lastly, a tool capable of phylogenetic classification such as checkM is used to calculate provisional phylogenetic classification information. Processing up to this point is preprocessing of partial base sequences of a single biological unit.

Once single biological unit genome analysis is completed, provisional phylogenetic classification in the draft genomic information table of a microorganism genome database is referenced to extract corresponding draft genomic information and genetic information. The marker type of genetic information is referenced to obtain an organism lineage identification sequence. A gene of the same protein family as the protein family of the organism lineage identification sequence is extracted from the genetic information in the single biological unit genomic data. If there is no corresponding genetic information, the processing ends to transition to the next processing. If there is corresponding genetic information, homology search is performed with a homology analysis tool such as BLAST on the gene base sequence in unit genomic data and organism lineage identification sequence in a round robin format. Since only pairs with homology at or above a certain threshold value are targeted, pairs at or below a certain threshold value (e.g., homology of 70% or less) are excluded. A gene base sequence in unit genomic data with the highest homology in each organism lineage identification sequence is detected. The weighted average of homology and matched base sequence length are found as the degree of similarity (distance) between two genomes. If a plurality of draft genomes with the same degree of similarity are detected, homology is searched between assembled base sequences, instead of with an organism lineage identification sequence, in a round robin format. The degree of similarity is calculated by performing the same processing as with an organism lineage identification sequence. The draft genome with the highest degree of similarity is used as the baseline for clustering.

### (Example 4) Clustering of single biological unit

Figure **4** shows a method for creating a cluster of the same lineage, when there is no corresponding draft genome in a microorganism genome database, in newly measured single biological unit genomic data. This Example is described while assuming that each single biological unit genomic data (partial base sequence, genomic information, assembled base sequence, provisional phylogenetic classification, genetic information, and gene base sequence) is already obtained by the preliminary processing shown in Example 2. Single biological unit genomic data for the same lineage is clustered by provisional phylogenetic classification. Phylogenetic classification is determined by processing an assembled base sequence by using a tool such as checkM. Meanwhile, an assembled base sequence of a single biological unit often does not cover the entire genome, so that phylogenetic classification is often crude, and a cluster containing various lineages or organisms is likely created. In this regard, whether single biological unit genomic data within a cluster can be subdivided using an organism lineage identification sequence registered in a microorganism genome database is evaluated. Figure **6** shows a method of subdividing single biological unit genomic data considered to be of a cluster of the same lineage. The marker type of the genetic information table registered in a microorganism genome database is referenced to obtain an organism lineage identification sequence and a corresponding protein family. The degree of similarity is calculated for single biological unit genomic data within a cluster by using an organism lineage identification sequence in a round robin format by the same method as the method of finding the degree of similarity between genomes shown in Example 2. A similarity matrix found in this regard is read in with statistical processing software R or the like to perform network analysis. When R is used, the igraph function can be utilized as a network analysis function. A community is then detected from an output of the network analysis. For community detection in R, a method based on edge betweenness centrality, method based on random walk, method based on a greedy algorithm, method based on a unique vector, method based on multistage optimization, method based on spin glass theory, method based on label propagation, method based on Infomap, or the like is implemented. Single biological unit genomic data is subdivided based on the detected community. An example using network analysis and community detection for subdivision was shown, but a method of subdivision using hierarchical (nonhierarchical) clustering is also conceivable. However, in such a case, the degree of similarity would be a missing value if there is no common organism lineage identification sequence for calculating the degree of similarity between two single biological unit genomic data, so that clustering analysis would not be able to be performed. For this reason, it is understood that use of network analysis and community detection that are compatible even with a missing value is rather preferable.

### (Example 5) Identification of new organism lineage identification sequence

Figure **5** shows a method of identifying a new organism lineage identification sequence for performing phylogenetic classification from genetic information of a draft genome registered in a microorganism genome database. A frequency table of gene families corresponding to draft genomes shown in Figure **5** is created using draft genome genetic information and draft genomic information registered in a microorganism genome database. The cells showing 1 in the frequency table indicate a single copy gene within the draft genome. Since an organism lineage identification sequence should be a single copy gene, cells that are not for a single copy gene are ignored. The ratio of single copy genes in the entire draft genome is calculated in each protein family. Since it is better as an organism lineage identification sequence if more of the draft genome is covered by single copy genes, protein families are sorted in descending order of the ratio of single copy genes, and a protein family meeting a baseline value (e.g., single copy genes are present in the draft genome at 90% or higher) is re-identified as an organism lineage identification sequence. The protein families can also be sorted in descending order of the ratio of single copy genes to use any number of top ranking protein families as an organism lineage identification sequence. Further, an organism lineage identification sequence known in bacteria or the like from previous studies can be used with the organism lineage identification sequence identified above.

It is also understood that the method proposed in D. H. Parks, et. al., 2015 can also be applied as a method of creating an organism lineage identification sequence that is different from the method described above. This is a method of creating a phylogenetic tree of the draft genome and defining an organism lineage identification sequence for each node, which is used as input data for checkM.

### (Example 6) Optimal draft genome construction

Figure **7** shows a method of constructing an optimal draft genome. Single biological unit genomic data is clustered as the same lineage by Examples 2 and 3. Since Example 2 performs clustering based on a draft genome of a microorganism genome database, a partial base sequence of a single biological unit genome corresponding to the draft genome is obtained from the microorganism genome database and added to the cluster. Single biological unit genomic data within the cluster is sorted with respect to a baseline value. In this regard, the data is sorted in descending order of contamination percentage. However, single biological unit genomic data that does not meet a certain level is excluded. In this regard, single biological unit genomic data with a completion percentage that is less than 10% is excluded. The two highest ranking single biological unit genomic data are then extracted to create a provisional set for draft genome construction. Three next highest ranking single biological unit genomic data are extracted to similarly create a provisional set for draft genome construction. In this manner, single biological unit genomic data is sequentially added in order of the highest ranking to create sets for provisional draft genome construction, where the number of sets is one less than the number of single biological unit genomic data within the cluster. Cleaning and Co-assembly of a Single-Cell Amplified Genome (ccSAG) proposed in M. Kogawa, et al., 2018 is applied to these sets for provisional draft genome construction to construct a provisional draft genome. The provisional draft genome can be expressed as a function using the number of single biological unit genomic data used for construction as an explanatory variable and a draft genome evaluation value such as the completion percentage or contamination percentage as an objective variable. This allows determination of whether the draft genome has converged so that improvement in quality can no longer be expected even by adding single biological unit genomic data or the like. With a draft genome without expectation of improvement in quality, improved speed in processing can be expected by forgoing construction of an optimal draft genome or the like. The highest quality draft genome among the provisional draft genome and existing draft genome is selected. If this is not an existing draft genome, the draft genome in a microorganism genome database is updated. Single biological unit genomic data that has been newly identified as a cluster is registered in single biological unit genomic data in the microorganism genome database.

### (Example 7) Finishing of draft genome

Figure **8** shows a method of finishing a draft genome registered in a microorganism genome database. A converged draft genome can be extracted from a microorganism genome database by using a function for evaluating convergence of a draft genome created in Example 5. Such a converged draft genome is subjected to finishing. There are two patterns of finishing methods. First is a method using single biological unit genomic data corresponding to the extracted draft genome. Extracted single biological unit genomic data is sorted with respect to a certain baseline value. In this regard, data is sorted in descending order of contamination percentage. However, single biological unit genomic data that does not meet a certain level is excluded. In this regard, single biological unit genomic data with a completion percentage that is less than 10% is excluded. ccSAG with changed parameters is performed using all single biological unit genomic data meeting a baseline. A parameter that can detect a longer assembled base sequence is set as the parameter. The draft genome created in this regard is the first finishing draft genome. Second is detection of a bridge assembled base sequence that is linked to an assembled base sequence of a draft genome among assembled base sequences of all single biological unit genomic data that meet a baseline. Homology is analyzed on the assembled base sequence of the draft genome and assembled base sequences of all single biological unit genomic data that meet the baseline in a round robin format with a homology analysis tool such as BLAST. If a result where one ends of two assembled base sequences of a draft genome match both ends of one assembled base sequence of single biological unit genomic data is obtained, the assembled base sequence of single biological unit genomic data plays a role of linking the draft genome assembled base sequences. A draft genome connected by an assembled base sequence that serves as a bridge in this manner is the second finishing draft genome. First and second finishing draft genomes are compared with a registered draft genome with respect to a baseline value, and the highest quality draft genome is selected. If the selected draft genome is not an existing draft genome, the draft genome of a microorganism genome database is updated.

### (Example 8) Subdivision of draft genome

Figure **9** shows a method of subdividing draft genomes registered in a microorganism genome database by lineages. A converged draft genome can be extracted from a microorganism genome database with a function for evaluating convergence of a draft genome created in Example 5. Such a converged draft genome is subjected to subdivision. Single biological unit genomic data corresponding to the extracted draft genome is sorted with respect to a certain baseline value. In this regard, data is sorted in descending order of contamination percentage. However, single biological unit genomic data that does not meet a certain level is excluded. In this regard, single biological unit genomic data with a completion percentage that is less than 10% is excluded. All single biological unit genomic data that meets a baseline is subjected to the subdivision in Figure **6** performed in Example 3. The optimal draft genome construction in Figure **7** is performed on each subdivided cluster to obtain the optimal draft genome of each cluster. The plurality of optimal draft genomes and registered draft genome are compared with respect to a certain baseline value, and the higher quality draft genome therebetween is selected. If the selected draft genome is not an existing draft genome, the existing draft genome is deleted from a microorganism genome database, and the subdivided draft genome is newly registered.

### (Example 9) Addition of sequence to external database

A higher quality genome can be constructed as shown in Figure **12** by utilizing an external independent database. If, for example, SAGs of 1 to 4 are the data of the project, the genome of strain 2 is constructed from only one SAG. In this regard, if external project data such as 5 and 6 can be added, a draft genome can be constructed from three SAG for strain 2. Thus, a higher quality genome can be constructed.

### (Example 10) Bias homogenization processing Objective and method

(Amplification) Bias homogenization is performed to improve the quality of a genome sequence obtained by assembly of sequence data including a bias. Specifically, a certain amount of sequence reads of a sequence site found to have a large number of duplications is removed based on results of mapping sequence reads to a reference genome sequence to correct a bias in the sequence reads for homogenization (Figure **13****).**

For the reference genome sequence, the genome of a known relative organism species or a DNA sequence created by assembly of sequence data itself on which bias homogenization is performed can be used. The resulting draft genome complement ratio or sequence fragment count is improved by assembly of sequence data that has been homogenized. Depending on the situation, further improvement in genome quality is expected by repeated homogenization using a genome sequence created from homogenized sequence data as a reference sequence. Specifically, the following was performed.

A genome was assembled using nanopore sequence data (GridION) on E. coli K12 strain single cell amplified genome (SAG). Sequence data with significantly different read depth for each genome region (Figure **14****,** top line chart) was directly used and assembled. As a result, a genome sequence on a region exhibiting a relatively low read depth was not acquired (Figure **14****,** gap in the bottom strip). Subsequently, sequence data was mapped using the assembled genome sequence as a reference sequence, and selective reads were removed so that the maximum read depth would be 100× for each region to homogenize the sequence data (Figure **15****,** top line chart). When the homogenized sequence data was reassembled, sequence construction of a genome region that was not obtained in the initial assembly was confirmed (Figure **15****,** bottom strip). Furthermore, improved genome complement ratio and reduction in genome sequence fragment count were also confirmed by repeating homogenization-assembly by using the newly assembled genome sequence as a reference sequence (Table 1).

**[Table 1]**

| Table 1 Evaluation of number of homogenization-assembly loops and acquired genome sequence | | | | | |
|---|---|---|---|---|---|
| Homogenization-assembly loop | Loop 0 | Loop 1 | Loop 2 | Loop 3 | Loop 4 |
| Sequence fragment count | 52 | 51 | 11 | 8 | 7 |
| E. coli genome complement ratio (%) | 70.539 | 97.224 | 98.909 | 98.845 | 98.916 |

### (Note)

As described above, the present disclosure is exemplified by the use of its preferred embodiments. However, it is understood that the scope of the present disclosure should be interpreted solely based on the Claims. It is also understood that any patent, any patent application, and any references cited herein should be incorporated herein by reference in the same manner as the contents are specifically described herein. The present application claims priority to Japanese Patent Application No. 2019-85839 filed on April 26, 2019 with the Japan Patent Office. It is understood that the entire content thereof is incorporated herein by reference in the same manner as if the contents are specifically described herein.

### [Industrial Applicability]

Automation of processing of single cell data of microorganisms and the like is enabled.

## Claims

1. A method of giving an instruction to a computer to execute processing of sequence information on a single biological unit, the computer given the instruction executing:
(A) a step of clustering partial sequence information of sequence information on a plurality of single biological units for each of the same lineages based on an organism lineage identification sequence; and
(C) a step of creating a sequence information draft for the single biological units by using the partial sequence information of sequence information on a single biological units and sequence information on the single biological units in a database created independently from the clustering.

2. The method of claim 1, further comprising:
(B) a step of adding, to the cluster, partial sequence information on the single biological units corresponding to the cluster in a database.

3. The method of claim 1 or 2, wherein (C) comprises removing a certain amount of partial sequence information comprising a sequence site found to have a large number of duplications to correct a bias in sequence reads.

4. A method of giving an instruction to a computer to execute screening of candidates of an organism lineage identification sequence, the computer given the instruction executing:
A) a step of extracting genes without duplication from a draft in a database;
B) a step of calculating the number or a ratio of single copy genes for each of the genes; and
C) a step of selecting a gene with the number or ratio of single copy genes greater than or equal to a predetermined value as a candidate of an organism lineage identification sequence.

5. A method of giving an instruction to a computer to execute processing of sequence information on a single biological unit, the computer given the instruction executing:
(D) a step of ranking partial sequence information of sequence information on a plurality of single biological units from a highest quality based on a predetermined judgment criterion;
(E) a step of selecting a population of a predetermined number of pieces of partial sequence information of sequence information on the plurality of single biological units from a highest ranking based on the ranking to construct a draft with a greater length than the partial sequence information from the partial sequence information; and
(E') a step of selecting a population of a set of a different number of pieces of partial sequence information of sequence information on the single biological units from the population, constructing a draft with a greater length than the partial sequence information from the partial sequence information, and selecting a draft created up to this point based on a predetermined judgment criterion.

6. The method of giving an instruction to a computer to execute processing of sequence information on a single biological unit of claim 5, the computer given the instruction executing:
(F) a step of comparing the selected draft with partial sequence information of sequence information on a single biological unit that was not selected in (E) or (E') and selecting partial sequence information of sequence information on the single biological unit having a sequence of a portion that is not included in the draft;
(G) a step of creating a longer draft by using the sequence information selected in (F) and the selected draft;
(G') optionally, a step of repeating (G) until the longer draft reaches a full length of sequence information; and
(G") optionally, a step of repeating the steps of claim 5 based on a judgment criterion with a lower criterion in the entire partial sequence information constituting the draft.

7. A method of giving an instruction to a computer to execute processing of sequence information on a single biological unit, the computer given the instruction executing:
(A) a step of clustering partial sequence information of sequence information on a plurality of single biological units for each of the same lineages based on an organism lineage identification sequence;
(H) a step of evaluating partial sequence information of sequence information on the plurality of single biological units constituting sequence information on a single biological unit based on an organism lineage identification sequence within the cluster of the same lineage, and reclustering within the cluster of the same lineage;
(H') a step of comparing a sequence information draft created from the cluster of the same lineage with a sequence information draft created from the reclustered cluster; and (I) a step of determining whether reclustering in (H) is appropriate for a result of comparison based on a predetermined judgment criterion, and, if appropriate, registering the draft in a database as a new group.

8. The method of claim 7, wherein the reclustering is performed through network analysis and community detection.

9. A method of giving an instruction to a computer to execute processing of sequence information on a single biological unit, the computer given the instruction executing:
**(A)** a step of clustering partial sequence information of sequence information on a plurality of single biological units for each of the same lineages based on an organism lineage identification sequence;
(D) a step of ranking partial sequence information of sequence information on the plurality of single biological units belonging to the cluster of the same lineage from a highest quality based on a predetermined judgment criterion;
(E) a step of selecting a population of a predetermined number of pieces of partial sequence information of sequence information on the plurality of single biological units from a highest ranking based on the ranking to construct a draft with a greater length than the partial sequence information from the partial sequence information;
(E") a step of selecting a population of a set of a different number of pieces of partial sequence information of sequence information on the single biological unit from the population, constructing a draft with a greater length than the partial sequence information from the partial sequence information, and evaluating a draft created up to this point based on a predetermined judgment criterion;
(H) a step of evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change due to an increase in the number in a population of a set of sequence information;
(H') a step of comparing a sequence information draft created from the cluster of the same lineage with a sequence information draft created from the reclustered cluster; and (J) a step of determining whether reclustering in (H) is appropriate for a result of comparison based on a predetermined judgment criterion, and, if appropriate, repeating (D) to (E') for partial sequence information of sequence information on the plurality of single biological units belonging to the reclustered cluster.

10. The method of any one of claims 1 to 3 and 5 to 9, wherein the partial sequence information is determined by long-read sequencing.

11. A program for implementing a method of processing sequence information on a single biological unit on a computer, the method comprising:
(A) a step of clustering partial sequence information of sequence information on a plurality of single biological units for each of the same lineages based on an organism lineage identification sequence; and
(C) a step of creating a sequence information draft for the single biological units by using the partial sequence information of sequence information on the single biological units and sequence information on the single biological units in a database created independently from the clustering.

12. The program of claim 11, further comprising:
(B) a step of adding, to the cluster, partial sequence information on the single biological units corresponding to the cluster in the database.

13. The program of claim 11 or 12, wherein (C) comprises removing a certain amount of partial sequence information comprising a sequence site found to have a large number of duplications to correct a bias in sequence reads.

14. A program for implementing a method of screening candidates of an organism lineage identification sequence on a computer, the method comprising:
A) a step of extracting genes without duplication from a draft in a database;
B) a step of calculating the number or a ratio of single copy genes for each of the genes; and
C) a step of selecting a gene with the number or ratio of single copy genes greater than or equal to a predetermined value as a candidate of an organism lineage identification sequence.

15. A program for implementing a method of processing sequence information on a single biological unit on a computer, the method comprising:
(D) a step of ranking partial sequence information of sequence information on a plurality of single biological units from a highest quality based on a predetermined judgment criterion;
(E) a step of selecting a population of a predetermined number of pieces of partial sequence information of sequence information on the plurality of single biological units from a highest ranking based on the ranking to construct a draft with a greater length than the partial sequence information from the partial sequence information; and
(E') a step of selecting a population of a set of a different number of pieces of partial sequence information of sequence information on the single biological units from the population, constructing a draft with a greater length than the partial sequence information from the partial sequence information, and selecting a draft created up to this point based on a predetermined judgment criterion.

16. The program for implementing a method of processing sequence information on a single biological unit on a computer of claim 15, the method comprising:
(F) a step of comparing the selected draft with partial sequence information of sequence information on a single biological unit that was not selected in (E) or (E') and selecting partial sequence information of sequence information on the single biological unit having a sequence of a portion that is not included in the draft;
(G) a step of creating a longer draft by using the sequence information selected in (F) and the selected draft;
(G') optionally, a step of repeating (G) until the longer draft reaches a full length of sequence information; and (G") optionally, a step of repeating the steps of claim 15 based on a judgment criterion with a lower criterion in the entire partial sequence information constituting the draft.

17. A program for implementing a method of processing sequence information on a single biological unit on a computer, the method comprising:
(A) a step of clustering partial sequence information of sequence information on a plurality of single biological units for each of the same lineages based on an organism lineage identification sequence;
(H) a step of evaluating partial sequence information of sequence information on the plurality of single biological units constituting sequence information on a single biological unit based on an organism lineage identification sequence within the cluster of the same lineage, and reclustering within the cluster of the same lineage;
(H') a step of comparing a sequence information draft created from the cluster of the same lineage with a sequence information draft created from the reclustered cluster; and (I) a step of determining whether reclustering in (H) is appropriate for a result of comparison based on a predetermined judgment criterion, and, if appropriate, registering the draft in a database as a new group.

18. The program of claim 17, wherein the reclustering is performed through network analysis and community detection.

19. A program for implementing a method of processing sequence information on a single biological unit on a computer, the method comprising:
(A) a step of clustering partial sequence information of sequence information on a plurality of single biological units for each of the same lineages based on an organism lineage identification sequence;
(D) a step of ranking partial sequence information of sequence information on the plurality of single biological units belonging to the cluster of the same lineage from a highest quality based on a predetermined judgment criterion;
(E) a step of selecting a population of a predetermined number of pieces of partial sequence information of sequence information on the plurality of single biological units from a highest ranking based on the ranking to construct a draft with a greater length than the partial sequence information from the partial sequence information;
(E") a step of selecting a population of a set of a different number of pieces of partial sequence information of sequence information on the single biological unit from the population, constructing a draft with a greater length than the partial sequence information from the partial sequence information, and evaluating a draft created up to this point based on a predetermined judgment criterion;
(H) a step of evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change due to an increase in the number in a population of a set of sequence information;
(H') a step of comparing a sequence information draft created from the cluster of the same lineage with a sequence information draft created from the reclustered cluster; and (J) a step of determining whether reclustering in (H) is appropriate for a result of comparison based on a predetermined judgment criterion, and, if appropriate, repeating (D) to (E') for partial sequence information of sequence information on the plurality of single biological units belonging to the reclustered cluster.

20. The program of any one of claims 11 to 13 and 15 to 19, wherein the partial sequence information is determined by long-read sequencing.

21. A recording medium storing a program for implementing a method of processing sequence information on a single biological unit on a computer, the method comprising:
(A) a step of clustering partial sequence information of sequence information on a plurality of single biological units for each of the same lineages based on an organism lineage identification sequence; and
(C) a step of creating a sequence information draft for the single biological units by using the partial sequence information of sequence information on the single biological units and sequence information on the single biological units in a database created independently from the clustering.

22. The recording medium of claim 21, further comprising:
(B) a step of adding, to the cluster, partial sequence information on the single biological units corresponding to the cluster in the database.

23. The recording medium of claim 21 or 22, wherein (C) comprises removing a certain amount of partial sequence information comprising a sequence site found to have a large number of duplications to correct a bias in sequence reads.

24. A recording medium storing a program for implementing a method of screening candidates of an organism lineage identification sequence on a computer, the method comprising:
A) a step of extracting genes without duplication from a draft in a database;
B) a step of calculating the number or a ratio of single copy genes for each of the genes; and
C) a step of selecting a gene with the number or ratio of single copy genes greater than or equal to a predetermined value as a candidate of an organism lineage identification sequence.

25. A recording medium storing a program for implementing a method of processing sequence information on a single biological unit on a computer, the method comprising:
(D) a step of ranking partial sequence information of sequence information on a plurality of single biological units from a highest quality based on a predetermined judgment criterion;
(E) a step of selecting a population of a predetermined number of pieces of partial sequence information of sequence information on the plurality of single biological units from a highest ranking based on the ranking to construct a draft with a greater length than the partial sequence information from the partial sequence information; and
(E') a step of selecting a population of a set of a different number of pieces of partial sequence information of sequence information on the single biological units from the population, constructing a draft with a greater length than the partial sequence information from the partial sequence information, and selecting a draft created up to this point based on a predetermined judgment criterion.

26. The recording medium storing a program for implementing a method of processing sequence information on a single biological unit on a computer of claim 25, the method comprising:
(F) a step of comparing the selected draft with partial sequence information of sequence information on a single biological unit that was not selected in (E) or (E') and selecting partial sequence information of sequence information on the single biological unit having a sequence of a portion that is not included in the draft;
(G) a step of creating a longer draft by using the sequence information selected in (F) and the selected draft;
(G') optionally, a step of repeating (G) until the longer draft reaches a full length of sequence information; and
(G") optionally, a step of repeating the steps of claim 25 based on a judgment criterion with a lower criterion in the entire partial sequence information constituting the draft.

27. A recording medium storing a program for implementing a method of processing sequence information on a single biological unit on a computer, the method comprising:
(A) a step of clustering partial sequence information of sequence information on a plurality of single biological units for each of the same lineages based on an organism lineage identification sequence;
(H) a step of evaluating partial sequence information of sequence information on the plurality of single biological units constituting sequence information on a single biological unit based on an organism lineage identification sequence within the cluster of the same lineage, and reclustering within the cluster of the same lineage;
(H') a step of comparing a sequence information draft created from the cluster of the same lineage with a sequence information draft created from the reclustered cluster; and (I) a step of determining whether reclustering in (H) is appropriate for a result of comparison based on a predetermined judgment criterion, and, if appropriate, registering the draft in a database as a new group.

28. The recording medium of claim 27, wherein the reclustering is performed through network analysis and community detection.

29. A recording medium storing a program for implementing a method of processing sequence information on a single biological unit on a computer, the method comprising:
(A) a step of clustering partial sequence information of sequence information on a plurality of single biological units for each of the same lineages based on an organism lineage identification sequence;
(D) a step of ranking partial sequence information of sequence information on the plurality of single biological units belonging to the cluster of the same lineage from a highest quality based on a predetermined judgment criterion;
(E) a step of selecting a population of a predetermined number of pieces of partial sequence information of sequence information on the plurality of single biological units from a highest ranking based on the ranking to construct a draft with a greater length than the partial sequence information from the partial sequence information;
(E") a step of selecting a population of a set of a different number of pieces of partial sequence information of sequence information on the single biological unit from the population, constructing a draft with a greater length than the partial sequence information from the partial sequence information, and evaluating a draft created up to this point based on a predetermined judgment criterion;
(H) a step of evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change due to an increase in the number in a population of a set of sequence information;
(H') a step of comparing a sequence information draft created from the cluster of the same lineage with a sequence information draft created from the reclustered cluster; and (J) a step of determining whether reclustering in (H) is appropriate for a result of comparison based on a predetermined judgment criterion, and, if appropriate, repeating (D) to (E') for partial sequence information of sequence information on the plurality of single biological units belonging to the reclustered cluster.

30. The recording medium of any one of claims 21 to 23 and 25 to 29, wherein the partial sequence information is determined by long-read sequencing.

31. A system for processing sequence information on a single biological unit, the system comprising:
(A) a clustering unit for clustering partial sequence information of sequence information on a plurality of single biological units for each of the same lineages based on an organism lineage identification sequence; and
(C) a draft creation unit for creating a sequence information draft for the single biological units by using the partial sequence information of sequence information on the single biological units and sequence information on the single biological units in a database created independently from clustering by the clustering unit of (A).

32. The system of claim 31, further comprising:
(B) an additional information addition unit for adding, to the cluster, partial sequence information on the single biological units corresponding to the cluster in the database.

33. The system of claim 31 or 32, wherein (C) comprises removing a certain amount of partial sequence information comprising a sequence site found to have a large number of duplications to correct a bias in sequence reads.

34. A system for screening candidates of an organism lineage identification sequence, the system comprising:
A) an extraction unit for extracting genes without duplication from a draft in a database;
B) a calculation unit for calculating the number or a ratio of single copy genes for each of the genes; and
C) a selection unit for selecting a gene with the number or ratio of single copy genes greater than or equal to a predetermined value as a candidate of an organism lineage identification sequence.

35. A system for processing sequence information on a single biological unit, the system comprising:
(D) a ranking unit for ranking partial sequence information of sequence information on the plurality of single biological units from a highest quality based on a predetermined judgment criterion;
(E) a draft construction unit for selecting a population of a predetermined number of pieces of partial sequence information of sequence information on the plurality of single biological units from a highest ranking based on the ranking to construct a draft with a greater length than the partial sequence information from the partial sequence information; and
(E') a selection unit for selecting a population of a set of a different number of pieces of partial sequence information of sequence information on the single biological units from the population, constructing a draft with a greater length than the partial sequence information from the partial sequence information, and selecting a draft created up to this point based on a predetermined judgment criterion.

36. The system for processing sequence information on a single biological unit of claim 35, the system comprising:
(F) a selection unit for comparing the selected draft with partial sequence information of sequence information on a single biological unit that was not selected in (E) or (E') and selecting partial sequence information of sequence information on the single biological unit having a sequence of a portion that is not included in the draft;
(G) a draft improvement unit for creating a longer draft by using the sequence information selected in (F) and the selected draft;
(G') optionally, a draft construction unit for repeating draft creation in (G) until the longer draft reaches a full length of sequence information; and
(G") optionally, means for repeating the ranking, draft construction, and selection of (D), (E), and (E') of claim 35 based on a judgment criterion with a lower criterion in the entire partial sequence information constituting the draft.

37. A system for processing sequence information on a single biological unit, the system comprising:
(A) a clustering unit for clustering partial sequence information of sequence information on a plurality of single biological units for each of the same lineages based on an organism lineage identification sequence;
(H) a reclustering unit for evaluating partial sequence information of sequence information on the plurality of single biological units constituting sequence information on a single biological unit based on an organism lineage identification sequence within the cluster of the same lineage, and reclustering within the cluster of the same lineage;
(H') a comparison unit for comparing a sequence information draft created from the cluster of the same lineage with a sequence information draft created from the reclustered cluster; and
(I) a registration unit for determining whether reclustering in (H) is appropriate for a result of comparison based on a predetermined judgment criterion, and, if appropriate, registering the draft in a database as a new group.

38. The system of claim 37, wherein the reclustering unit performs reclustering through network analysis and community detection.

39. A system for processing sequence information on a single biological unit, the system comprising:
(A) a clustering unit for clustering partial sequence information of sequence information on a plurality of single biological units for each of the same lineages based on an organism lineage identification sequence;
(D) a ranking unit for ranking partial sequence information of sequence information on the plurality of single biological units belonging to the cluster of the same lineage from a highest quality based on a predetermined judgment criterion;
(E) a draft construction unit for selecting a population of a predetermined number of pieces of partial sequence information of sequence information on the plurality of single biological units from a highest ranking based on the ranking to construct a draft with a greater length than the partial sequence information from the partial sequence information,
(E") selecting a population of a set of a different number of pieces of partial sequence information of sequence information on the single biological unit from the population, constructing a draft with a greater length than the partial sequence information from the partial sequence information, and evaluating a draft created up to this point based on a predetermined judgment criterion;
(H) a reclustering unit for evaluating partial sequence information of sequence information on the plurality of single biological units constituting sequence information on a single biological unit based on an organism lineage identification sequence within the cluster of the same lineage, and reclustering within the cluster of the same lineage, if evaluation of the draft does not change due to an increase in the number in a population of a set of sequence information;
(H') a comparison unit for comparing a sequence information draft created from the cluster of the same lineage with a sequence information draft created from the reclustered cluster; and
(J) means for determining whether reclustering in (H) is appropriate for a result of comparison based on a predetermined judgment criterion, and, if appropriate, repeating (D) to (E') for partial sequence information of sequence information on the plurality of single biological units belonging to the reclustered cluster.

40. The system of any one of claims 31 to 33 and 35 to 39, wherein the partial sequence information is determined by long-read sequencing.
